(19) Europäisches Patentamt
European Patent Office
Office européen des brevets

(11) **EP 3 715 844 A1**

(12) **EUROPEAN PATENT APPLICATION**
published in accordance with Art. 153(4) EPC

(43) Date of publication:
**30.09.2020 Bulletin 2020/40**

(21) Application number: **18878648.7**

(22) Date of filing: **20.11.2018**

(51) Int Cl.:
*G01N 33/48* (2006.01)        *A61B 6/00* (2006.01)
*A61B 6/03* (2006.01)        *A61K 49/04* (2006.01)

(86) International application number:
**PCT/JP2018/042826**

(87) International publication number:
**WO 2019/098385 (23.05.2019 Gazette 2019/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.11.2017   JP 2017222685
20.11.2017   JP 2017222686**

(71) Applicant: **Konica Minolta, Inc.
Tokyo 100-7015 (JP)**

(72) Inventors:
• **AIMIYA, Takuji
Tokyo 100-7015 (JP)**

• **FURUSAWA, Naoko
Tokyo 100-7015 (JP)**
• **NAKANO, Yasushi
Tokyo 100-7015 (JP)**
• **GONDA, Kohsuke
Sendai-shi
Miyagi 980-8577 (JP)**
• **HAMADA, Yoh
Sendai-shi
Miyagi 980-8577 (JP)**
• **TOKUNAGA, Masayuki
Sendai-shi
Miyagi 980-8577 (JP)**

(74) Representative: **Henkel & Partner mbB
Patentanwaltskanzlei, Rechtsanwaltskanzlei
Maximiliansplatz 21
80333 München (DE)**

(54) **DRUG EVALUATION METHOD**

(57)    The present invention relates to a method for evaluating a drug that is involved in angiogenesis. The method includes a drug administration step (A) in which the drug that is involved in angiogenesis is administered to a subject that has a lesion. The method also involves: performing an imaging step (1) in which radiography is performed on the subject after the blood vessels thereof have been made to contain metal nanoparticles or an imaging step (2) in which fluorescence staining and fluorescence imaging are performed using a dimeric protein; and evaluating the drug using an image acquired in imaging step (1) or (2).

**Description**

Technical Field

The present invention relates to a drug evaluation method.

Background Art

Recently, it has been revealed that angiogenesis plays a very important role in many diseases such as a solid cancer, diabetic retinopathy, and rheumatoid arthritis. For example, a tumor that is a solid cancer forms a new blood vessel so as to be able to supply nutrients and oxygen required for a rapid cell growth of the tumor. For this reason, an anticancer agent having an effect of inhibiting angiogenesis has been recently developed, and an antitumor effect on various types of tumors is expected.

For example, for angiogenesis, various factors such as a fibroblast growth factor (FGF), a vascular endothelial growth factor (VEGF), and a platelet-derived growth factor (PDGF) are involved. Among these factors, VEGF is known to be bonded to a VEGF receptor on a vascular endothelial cell, to regulate gene expression, vascular permeability, cell growth, and the like in the vascular endothelial cell, and to promote angiogenesis, and is an important target in development of an angiogenesis inhibitor.

For evaluation of development of a drug involved in angiogenesis including these factors and an effect of a candidate drug, it is important to detect and visualize a factor or a receptor to be a drug target, or a lesion itself.

For example, as a means for visualizing a lesion or an organ, a technique such as computed tomography (CT) for imaging, using a computer, a cross-sectional image obtained by scanning an object using radiation or the like is widely used in a clinical site. CT is a powerful medical diagnostic technique that can obtain three-dimensional images of a blood vessel and an organ inside a human body in a non-invasive manner. Diagnosis is performed by measuring a difference in radiation absorptivity between internal tissues, and using a difference in contrast between a lesion site and surrounding normal tissues as an indicator.

A contrast agent is usually used in order to improve a CT image contrast to enhance diagnostic accuracy. However, an iodine-based contrast agent currently commonly used in a hospital or the like has a risk of causing a side effect disadvantageously. Non Patent Literature 1 describes a method for detecting a tumor by performing CT using gold nanoparticles having various sizes and surface-modified with a polyethylene glycol chain as a contrast agent.

Efficacy of a candidate drug can be evaluated by visualizing not only the size of a lesion but also the lesion and a blood vessel around the lesion.

The method disclosed in Patent Literature 1 sacrifices a tumor-bearing model mouse to which an angiogenesis inhibitor has been administered, and evaluates an effect of the inhibitor on angiogenesis.

Patent Literature 2 describes a method for visualizing a microvascular system by magnetic resonance imaging (MRI) using a gadolinium complex as a contrast agent. Non Patent Literature 2 describes a method for visualizing a microvascular system by CT imaging using gold nanoparticles as a contrast agent.

For a VEGF receptor and other factors involved in angiogenesis, a detection method for performing DAB staining on a tissue section extracted from a lesion, and capturing an image in a bright field to perform visualization (imaging) has been widely used conventionally.

However, in order to accurately evaluate movement of a factor involved in angiogenesis, detection sensitivity of the factor is important. However, in the existing methods, the sensitivity and the staining degree vary depending on the skill of an operator and an environment disadvantageously, and furthermore, quantification is poor disadvantageously. Therefore, it is difficult to specify a detailed expression site of a factor involved in angiogenesis in a tissue, an expression level in each cell, or a difference in the expression level among cells. Therefore, recently, in order to detect VEGF in more detail, a new method using VEGF which is a VEGF ligand has been attempted. Non Patent Literature 3 detects a VEGF receptor using VEGF having an amino group modified with biotin carried on a streptavidin-bonded quantum dot as a probe. By using such a dimeric VEGF as a probe, a VEGF receptor can be precisely detected, and physiological activity thereof can be evaluated.

Citation List

Patent Literature

Patent Literature 1: JP 2007-526756 A
Patent Literature 2: US 2008/0294035 A

Non Patent Literature

Non Patent Literature 1: Nakagawa et al. Science and Technology of Advanced Materials, VOL. 17, NO. 1, 387-397, 2016
Non Patent Literature 2: Quan-Yu Cai et al. Investigative Radiology. Volume 42, Number 12, 797-806, December 2007
Non Patent Literature 3: Hamada et al., BLOOD, 29 SEPTEMBER 2011 VOLUME 118, NUMBER 13:e93-100

Summary of Invention

Technical Problem

As described above, in a case where a blood vessel is detected in evaluation of a drug involved in angiogenesis, the method disclosed in Patent Literature 1 requires sacrifice of a mouse, and cannot track a change of a blood vessel with days in the same mouse disadvantageously. Non Patent Literature 2 describes a method for visualizing a microvascular system by performing CT imaging using gold nanoparticles as a contrast agent, but does not describe measurement of a change of a blood vessel with days, use for evaluation of a drug, or the like, either.

The method disclosed in Patent Literature 2 uses magnetic resonance imaging (MRI) using a gadolinium complex as a contrast agent, but the method is a relatively laborious and time-consuming test method, and does not have sufficient spatial resolution.

Furthermore, in a case where a method for detecting a factor involved in angiogenesis is used in evaluation of a drug involved in angiogenesis, Non Patent Literature 3 discloses a method for staining a VEGF receptor. However, this method stains a cell in which a VEGF receptor is artificially expressed at a high level in a vascular endothelial cell, and there is a possibility that a cell having a normal or low VEGF receptor expression level cannot be stained or cannot be detected with sufficient sensitivity. In particular, in a case where the expression of a VEGF receptor in a vascular endothelial cell or a tumor cell is reduced by administration of a drug that inhibits angiogenesis, there is a high possibility that sufficient detection sensitivity for accurately evaluating drug efficacy cannot be obtained.

The present invention relates to a method for evaluating a drug involved in angiogenesis by observing an effect of the drug over time with sufficient accuracy.

Solution to Problem

In order to solve the above problems, the present inventor compared and observed a change in information including blood vessel information such as the size of a tumor or a blood vessel volume using an image acquired by performing radiography of a subject using metal nanoparticles, fluorescently staining a factor involved in angiogenesis using a dimeric protein, and imaging the factor. As a result, the present inventor has found that an effect of a drug involved in angiogenesis can be evaluated.

That is, the present invention provides the following drug evaluation methods.

[Clause 1]
A drug evaluation method to be performed using an animal having a lesion as a subject, the method comprising:

a drug administration step (A) of administering a drug involved in angiogenesis to the subject;
further performing the following imaging step (1) a plurality of times, at least one of which is performed after the drug administration step (A), or performing the following imaging step (2); and
further evaluating the drug using an image acquired in the imaging step (1) or (2).

Imaging step (1): Radiography is performed on a subject in a state where metal nanoparticles are present in a blood vessel.
Imaging step (2): Using a dimeric protein containing a first monomer which is a fusion peptide fused to a tag bonded to streptavidin at one end, and a second monomer,
at least two tissue sections are obtained from among a tissue section collected from the subject before the drug administration step (A), a tissue section collected from the subject at a first time point after the drug administration step (A), and a tissue section collected from the subject at a second time point which is a time point after the first time point, each of the sections is fluorescently stained, and each of the fluorescently stained samples is imaged.

[Clause 2]

The drug evaluation method according to claim 1, wherein the dimeric protein contains a first monomer which is a fusion peptide fused to a tag bonded to streptavidin at one end, and a second monomer fused to a tag bonded to a protein other than streptavidin at one end.

[Clause 3]

The drug evaluation method according to claim 1 or 2, wherein the second monomer is a fusion peptide fused to a tag bonded to a protein to other than streptavidin only at one end.

[Clause 4]

The drug evaluation method according to any one of claims 1 to 3, wherein both the first monomer and the second monomer are linear.

[Clause 5]

The drug evaluation method according to any one of claims 1 to 4, wherein

a peptide sequence other than the tag bonded to streptavidin in the first monomer has the same sequence as

a peptide sequence other than the tag bonded to a protein other than streptavidin in the second monomer.

[Clause 6]

The drug evaluation method according to any one of claims 1 to 5, wherein the dimeric protein is derived from a vascular endothelial cell growth factor (VEGF) or a platelet-derived growth factor (PDGF).

[Clause 7]

The drug evaluation method according to any one of claims 1 to 6, wherein the fluorescence staining is a method for staining a target protein to be specifically bonded to the dimeric protein by bonding the dimeric protein to a fluorescent label.

[Clause 8]

The drug evaluation method according to any one of claims 1 to 6, wherein the fluorescence staining is a method for staining a target protein to be specifically bonded to the dimeric protein by bonding the target protein to a fluorescence labelling probe in which a fluorescent label is bonded to the dimeric protein.

[Clause 9]

The drug evaluation method according to claim 1, wherein the metal nanoparticles are gold nanoparticles.

[Clause 10]

The drug evaluation method according to claim 7 or 8, wherein the fluorescent label is a fluorescent dye, a quantum dot, or a fluorescent dye-integrated nanoparticle bonded to streptavidin.

[Clause 11]

The drug evaluation method according to any one of claims 1 to 10, wherein in the imaging step (2), fluorescent dye staining which is staining using a fluorescent dye is further performed.

[Clause 12]

The drug evaluation method according to claim 11, wherein the fluorescent dye staining is staining specific to a vascular endothelial cell.

[Clause 13]

The drug evaluation method according to any one of claims 1 to 12, wherein in the imaging step (2), dye staining with a dye and bright field imaging are further performed.

[Clause 14]

A dimeric protein comprising a first monomer which is a fusion peptide fused to a tag bonded to streptavidin at one end, and a second monomer.

[Clause 15]

A dimeric protein comprising a first monomer which is a fusion peptide fused to a tag bonded to streptavidin at one end, and a second monomer fused to a tag bonded to a protein other than streptavidin at one end.

[Clause 16]

The dimeric protein according to claim 14 or 15, wherein both the first monomer and the second monomer are linear.

[Clause 17]

The dimeric protein according to claim 15, wherein

a peptide sequence other than the tag bonded to streptavidin in the first monomer has the same sequence as

a peptide sequence other than the tag bonded to a protein other than streptavidin in the second monomer.

[Clause 18]

The dimeric protein according to any one of claims 14 to 17, derived from a vascular endothelial cell growth factor (VEGF) or a platelet-derived growth factor (PDGF).

[Clause 19]

A method for producing a dimeric protein, comprising:

a purification step of mixing

a first monomer which is a fusion peptide fused to a tag bonded to streptavidin only at one end, and
a second monomer which is a fusion peptide fused to a tag bonded to a protein to other than streptavidin only at one end; and
purifying a dimeric protein containing the first monomer and the second monomer by removing contaminants from the obtained mixture.

[Clause 20]
The method for producing a dimeric protein according to claim 19, wherein the purification step includes a first purification step of performing purification using a first purification column carrying a carrier to be specifically bonded to a tag fused to the first monomer, and a second purification step of performing purification using a second purification column carrying a carrier to be specifically bonded to a tag fused to the second monomer.
[Clause 21]
A fluorescence labelling probe in which the fluorescent label is bonded to the dimeric protein according to any one of claims 14 to 18.
[Clause 22]
The fluorescence labelling probe according to claim 21, wherein the fluorescent label is a fluorescent dye, a quantum dot, or a fluorescent dye-integrated nanoparticle bonded to streptavidin.
[Clause 23]
A fluorescence staining liquid comprising the fluorescence labelling probe according to claim 21 or 22.
[Clause 24] A fluorescent labeling and staining method for staining a target protein to be specifically bonded to the dimeric protein containing a first monomer and a second monomer by bonding a fluorescent label to the dimeric protein according to any one of claims 14 to 18, wherein
the fluorescent label is a fluorescent dye, a quantum dot, or a fluorescent dye-integrated nanoparticle bonded to streptavidin.
[Clause 25] The fluorescent labeling and staining method according to claim 24, further comprising fluorescent dye staining.
[Clause 26] The fluorescent labeling and staining method according to claim 24 or 25, wherein the dye staining is staining specific to a vascular endothelial cell.
[Clause 27] A physiological activity evaluation method comprising the staining method according to any one of claims 24 to 26.
[Clause 28] The drug evaluation method according to any one of claims 1 to 13, wherein the drug is evaluated by acquiring information including blood vessel information from an image obtained in the imaging step (1) or (2).
[Clause 29] The drug evaluation method according to claim 28, wherein the blood vessel information includes information on a volume of a blood vessel in the lesion.
[Clause 30] The drug evaluation method according to claim 28 or 29, wherein the blood vessel information includes positional information of a blood vessel in the lesion.
[Clause 31] The drug evaluation method according to any one of claims 1 to 13 and 28 to 30, wherein the information further includes pathological information.
[Clause 32] The drug evaluation method according to claim 31, wherein the pathological information includes information on a volume of the lesion.
[Clause 33] The drug evaluation method according to claim 31 or 32, wherein the pathological information includes positional information of a lesion.
[Clause 34] The drug evaluation method according to any one of claims 1 to 13 and 28 to 33, wherein the lesion is a tumor part.
[Clause 35] The drug evaluation method according to any one of claims 1 to 13 and 28 to 34, wherein the drug is involved in angiogenesis.
[Clause 36] The drug evaluation method according to any one of claims 1 to 13 and 28 to 35, wherein the drug is an angiogenesis inhibitor.
[Clause 37] The drug evaluation method according to any one of claims 28 to 36, wherein
the information including blood vessel information includes information on a volume of a blood vessel in the lesion and a volume of the lesion, and
a value obtained by dividing the volume of the blood vessel in the lesion by the volume of the lesion is calculated, and a change in the value is observed.
[Clause 38] The drug evaluation method according to any one of claims 1 to 13 and 28 to 37, wherein the radiography in the imaging step (1) is three-dimensional radiography, and the imaging in the imaging step (2) is fluorescence imaging.
[Clause 39] The drug evaluation method according to any one of claims 1 to 13 and 28 to 38, wherein the subject is an experimental animal.

[Clause 40] The drug evaluation method according to any one of claims 1 to 13 and 28 to 39, wherein the subject is a cancer-bearing mouse.

[Clause 41] A drug evaluation system for performing the drug evaluation method according to any one of claims 1 to 13 and 28 to 40, the drug evaluation system comprising

an imaging device including at least one of a radiography device and a fluorescence imaging device, and an information processing device, wherein

the information processing device

receives an image acquired by the radiography device or the fluorescence imaging device, and

evaluates a drug involved in angiogenesis using the received image.

[Clause 42] The drug evaluation system according to claim 41 for performing the drug evaluation method according to any one of claims 1 to 13 and 28 to 40, the drug evaluation system comprising

an imaging device including at least one of a radiography device and a fluorescence imaging device, and an information processing device, wherein

the information processing device

receives an image acquired by the radiography device or the fluorescence imaging device, and

further evaluates a drug involved in angiogenesis using information including blood vessel information acquired from the received image.

[Clause 43] The drug evaluation system according to claim 41 or 42, wherein the imaging device further includes a device for performing bright field imaging.

[Clause 44] The drug evaluation system according to any one of claims 41 to 43, further comprising a display device that displays at least one of the image, the information including blood vessel information, the analysis result, and drug evaluation.

[Clause 45] The drug evaluation system according to any one of claims 41 to 44, wherein the information processing device further includes an information storage device that stores at least one of the image, the information including blood vessel information, the analysis result, and drug evaluation as data.

[Clause 46] A program for causing a computer to execute the drug evaluation method according to any one of claims 1 to 13 and 28 to 40.

Advantageous Effects of Invention

The evaluation method of the present invention can evaluate a drug involved in angiogenesis more accurately than a conventional method.

Brief Description of Drawings

Fig. 1 is a flowchart in a drug evaluation method of the present invention (in a case where imaging step (1) is performed).

Fig. 2 is a flowchart of an experimental process performed in Example 1.

Fig. 3 is a graph illustrating a) a change in body weight and b) a change in tumor volume due to administration of bevacizumab in a bevacizumab treatment group and a control group in Example 1.

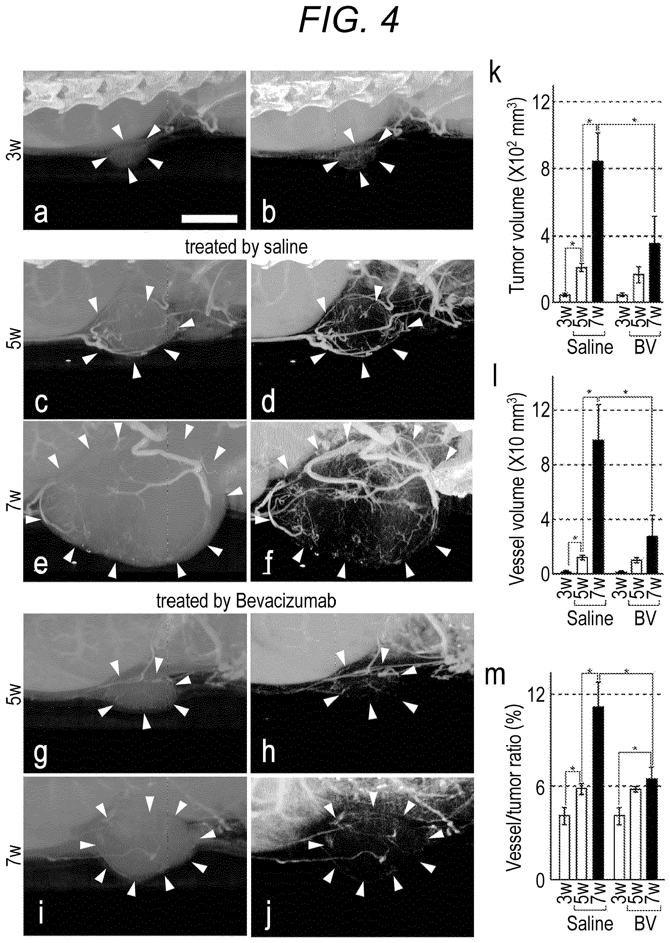

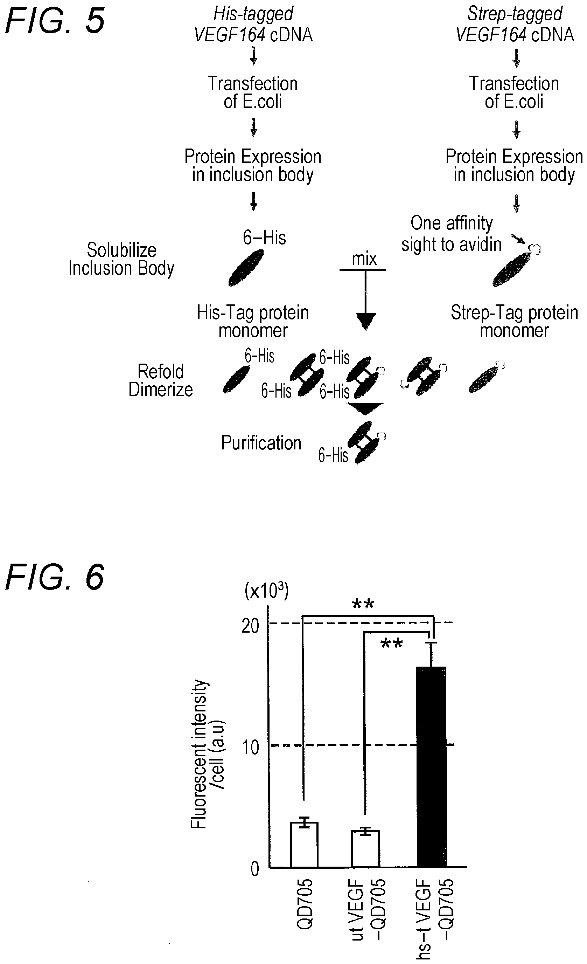

a to j of Fig. 4 illustrate radiation images of tumors and blood vessels, acquired by performing radiography on tumor parts of bevacizumab-treated and saline-treated mice three weeks, five weeks, and seven weeks after cancer cell transplantation. k to m of Fig. 5 are graphs illustrating a tumor volume, a blood vessel volume, and a ratio between the blood vessel volume and the tumor volume (blood vessel volume/tumor volume $\times$ 100 (%)) of bevacizumab-treated and saline-treated mice three weeks, five weeks, and seven weeks after cancer cell transplantation, respectively.

Fig. 5 is a flowchart illustrating a process for producing Strep-tag/His-tag-fused VEGF in Example 2.

Fig. 6 is a graph illustrating a result of quantification of fluorescent intensity measured in a staining result in Example 3. ut VEGF indicates a mouse VEGF164 recombinant protein, and hs-t VEGF indicates Strep-tag/His-tag-fused VEGF.

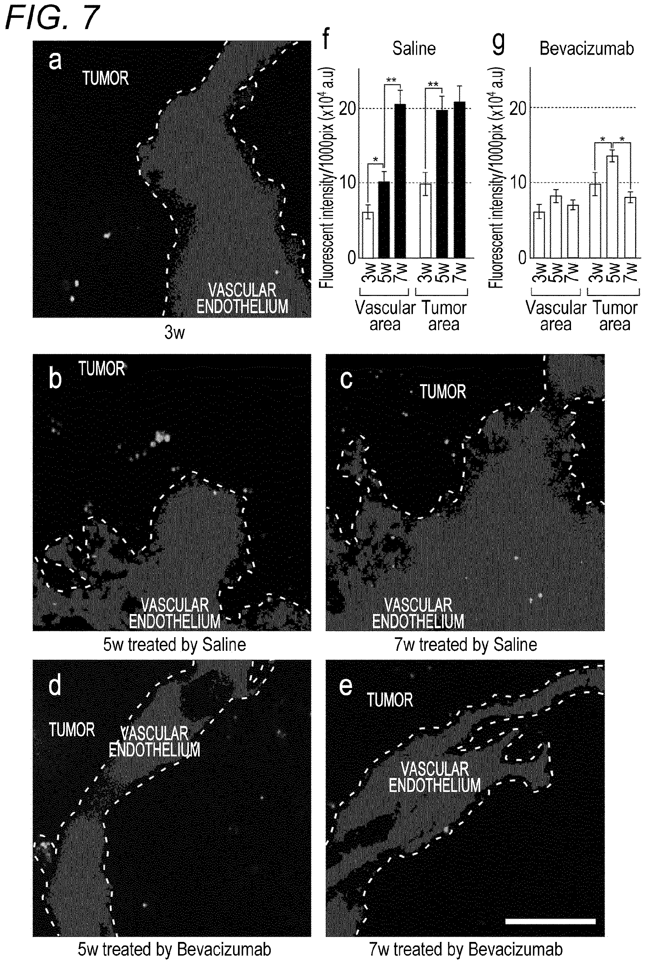

a) to e) of Fig. 7 are fluorescence images acquired in Example 4. The dotted line divides the image into a tumor area and a vascular endothelial area. f) and g) of Fig. 7 are graphs illustrating the expression levels of a VEGF receptor in the tumor cell area and the vascular endothelial area, calculated from fluorescence images acquired in the control group and the bevacizumab treatment group, respectively.

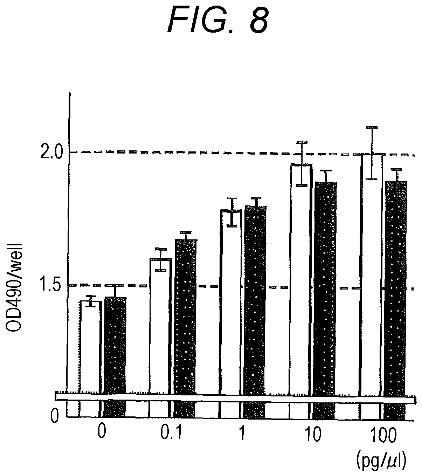

Fig. 8 illustrates a result of a cell growth test performed in Example 5.

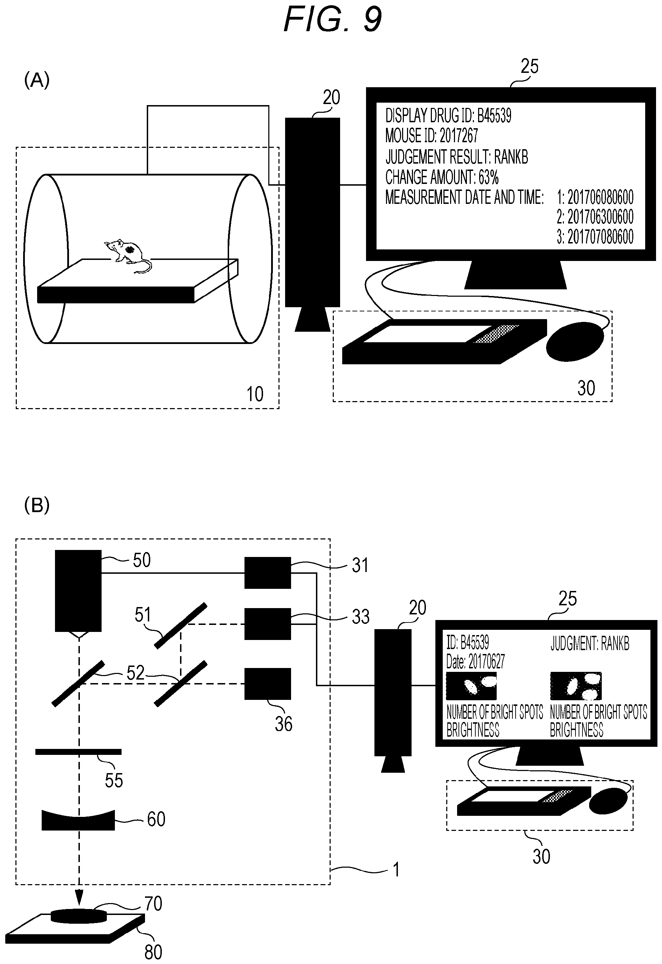

Fig. 9A is a schematic diagram of a system according to an embodiment of the present invention, the system including a radiography device as an imaging device. Fig. 9B is a schematic diagram of a system according to another embodiment of the present invention, the system including a fluorescence imaging device as an imaging device.

Description of Embodiments

A drug evaluation method of the present invention performs a drug administration step (A) of administering a drug involved in angiogenesis to a subject that is an animal having a lesion; performing the following imaging step (1) a plurality of times or performing the following imaging step (2); and
further evaluating the drug using an image acquired in the imaging step (1) or (2).
The drug evaluation method preferably evaluates the drug by acquiring information including blood vessel information described later from the image.
The drug evaluation method of the present invention may evaluate the drug only in an image acquired in the imaging steps (1), or may evaluate the drug only in an image acquired in the imaging step (2). In addition, by combining the drug evaluation method that has performed the imaging step (1) and the drug evaluation method that has performed the imaging step (2), in other words, by combining images acquired in the imaging step (1) and the imaging step (2), complex drug evaluation may be performed.

<Drug administration step (A)>

The drug administration step (A) according to the present invention is a step of administering a drug involved in angiogenesis to a subject described below. A lesion of the subject is not particularly limited, but for example, a tumor is preferably selected from a viewpoint of being involved in angiogenesis significantly.
The drug involved in angiogenesis is preferably an angiogenesis inhibitor, and more preferably an angiogenesis inhibitor used as an anticancer agent or a candidate drug thereof. The angiogenesis inhibitor is not particularly limited, but examples of the angiogenesis inhibitor used as the anticancer agent include bevacizumab (trade name: Avastin), sunitinib (trade name: Sutent), and sorafenib (trade name: Nexavar).
In the drug administration step (A), a drug administration form, a drug administration route, a drug administration period, the number of drug administrations, and the like to a subject are not particularly limited. By appropriately changing the drug administration form, the drug administration route, the drug administration period, the number of drug administrations, and the like and executing the evaluation method of the present invention a plurality of times, a drug can be evaluated in more detail.

<Imaging step (1)>

In the imaging step (1) according to the present invention, radiography is performed on a subject in a state where metal nanoparticles are present in a blood vessel. The radiography is preferably three-dimensional radiography.
In order to obtain the state where the metal nanoparticles are present in a blood vessel of a subject, for example, the metal nanoparticles are preferably injected into the blood vessel. A timing of injection into a blood vessel of a subject can be arbitrarily set as long as the metal nanoparticles can be present in the blood vessel during radiography. For example, each time when radiography is performed, the metal nanoparticles may be injected immediately before each radiography. Radiography may be performed a plurality of times in one injection of the metal nanoparticles.
In the drug evaluation method of the present invention, the imaging step (1) is performed a plurality of times, at least one of which is performed after the drug administration step (A).
The imaging step (1) needs to be performed at least once after the drug administration step (A). As long as the condition is satisfied, the imaging step (1) may be performed at a time point before the drug administration and at one or more time points after the drug administration, or at two or more time points after the drug administration. In the drug evaluation method of the present invention, each of the imaging step and an information acquiring step is preferably performed at one or more time points before drug administration and at two or more time points after the drug administration.
In a case where the imaging step (1) is performed after drug administration, a timing of performing the imaging step is not particularly limited, but preferably includes a sufficient time point such that an effect of the drug can be expected.

(Metal nanoparticles)

The metal nanoparticles used in the imaging step (1) are not particularly limited, but are preferably metal nanoparticles of silver, gold, platinum, and the like, and gold nanoparticles are more preferable from a viewpoint of low X-ray transmittance and excellent dispersibility. A dosage form when the metal nanoparticles are administered to a subject is not particularly limited, and only needs to be appropriately selected as needed. The amount of the metal nanoparticles to be injected can be freely selected according to the weight of a subject, the degree of a disease, and the like.

The metal nanoparticles are preferably surface-modified with an organic polymer or the like as necessary, and more preferably modified with polyethylene glycol (PEG), for example.

The average particle diameter of the metal nanoparticles is not particularly limited, but is preferably 1 to 300 nm, more preferably 1 to 150 nm, and still more preferably 1 to 50 nm.

The particle diameters of the metal nanoparticles can be determined by taking an electron micrograph using a transmission electron microscope (TEM). An average particle diameter of a group formed of a plurality of metal nanoparticles is determined by calculating particle diameters of a sufficient number (for example, 1000) of metal nanoparticles as described above, and then calculating an arithmetic average thereof. A coefficient of variation of the group formed of the plurality of metal nanoparticles is determined by calculating particle diameters of a sufficient number (for example, 1000) of metal nanoparticles as described above, and then performing a calculation of formula: $100 \times$ standard deviation of particle diameters/average particle diameter.

In the imaging step (1), the same subject, for example, a mouse can be imaged alive in each of the plurality of times. More detailed information on a blood vessel in a tumor part can be acquired because a tumor and a blood vessel volume are observed in the same subject with days, and an image with clearer contrast than a conventional method is acquired, for example. Furthermore, it is not necessary to sacrifice a subject such as a mouse when an effect of angiogenesis is evaluated, and an experiment can be performed using the same subject alive. Therefore, the number of subjects used in the experiment can be reduced advantageously.

<Imaging step (2)>

In the imaging step (2) according to the present invention, using a dimeric protein containing a first monomer which is a fusion peptide fused to a tag bonded to streptavidin at one end, and a second monomer, at least two tissue sections are obtained from among a tissue section collected from the subject before the drug administration step (A), a tissue section collected from the subject at a first time point after the drug administration step (A), and a tissue section collected from the subject at a second time point which is a time point after the first time point, each of the sections is fluorescently stained, and each of the fluorescently stained samples is imaged.

The dimeric protein used in the step (2) preferably contains a first monomer which is a fusion peptide fused to a tag bonded to streptavidin at one end, and a second monomer fused to a tag bonded to a protein other than streptavidin at one end.

(Fluorescence staining)

The fluorescence staining is a method for fluorescently staining a target protein to be specifically bonded to the dimeric protein, in which the target protein is preferably a factor involved in angiogenesis, and more preferably a protein involved in angiogenesis.

The fluorescence staining may be a method for staining the target protein by bonding the dimeric protein to a fluorescent label described below, or a method for staining the target protein by bonding the target protein to a fluorescence labelling probe (described later) in which a fluorescent label is bonded to the dimeric protein.

The fluorescent label is not particularly limited, but is preferably a fluorescent dye, a quantum dot, or a fluorescent dye-integrated nanoparticle bonded to streptavidin, and more preferably a fluorescent dye-integrated nanoparticle bonded to streptavidin.

Examples of the fluorescent dye include a fluorescent dye containing a low-molecular-weight organic compounds (those that are not high-molecular-weight organic compounds such as polymers), such as a fluorescein-based dye, a rhodamine-based dye, an Alexa Fluor (registered trademark, manufactured by Invitrogen)-based dye, a BODIPY (registered trademark, manufactured by Invitrogen)-based dye, a Cascade (registered trademark, Invitrogen)-based dye, a coumarin-based dye, an NBD (registered trademark)-based dye, a pyrene-based dye, a cyanine-based dye, a perylene-based dye, an oxazine-based dye, or a pyromethene-based dye. Among the dyes, a rhodamine-based dye such as Sulforhodamine 101 or TexasRed (registered trademark) that is a hydrochloride of Sulforhodamine 101, a perylene-based dye such as perylenediimide, and a pyromethene-based dye such as pyromethene 556 are preferable because of relatively high light resistance.

The quantum dot preferably has a shell around a particle dot containing a group II-VI compound, a group III-V compound, or a group IV compound, and may be surface-treated with an organic polymer or the like as necessary. For example, a commercially available product such as CdSe/ZnS (Invitrogen) having a particle surface modified with a carboxy group or CdSe/ZnS (Invitrogen) having a particle surface modified with an amino group may be used.

The phosphor-integrated particle is not particularly limited, but is a nano-sized particle (having a diameter of 1 $\mu$m or less) having a structure in which a plurality of fluorescent dyes or quantum dots is fixed and integrated inside or on a surface of a parent particle formed of an organic or inorganic substance, and is preferably such a particle that one particle can emit fluorescence with sufficient brightness.

Phosphor-integrated nanoparticles modified with streptavidin can be prepared, for example, as follows. A functional group is introduced by a reagent for introducing a functional group into each of the phosphor-integrated nanoparticle and streptavidin, and streptavidin and the phosphor-integrated nanoparticle are bonded to each other via a bond between the functional groups. A linker may be interposed between the functional groups. Examples of a combination of the functional groups include a combination of NHS ester group-amino group and a combination of thiol group-maleimide group. Examples of the linker include a linker such as N-[epsilon-Maleimidocaproyloxy] succinimide ester (EMCS) (Thermo Scientific Co., Ltd.).

<Fluorescent dye staining>

In the imaging step (2), fluorescent dye staining which is staining using a fluorescent dye can be further performed together. The fluorescent dye is preferably a fluorescent dye, a quantum dot, a phosphor-integrated nanoparticle, or the like other than the dimeric protein, the fluorescence labelling probe, or a fluorescence staining liquid described later.

A method of fluorescent dye staining is not particularly limited, and a known means such as immunofluorescence staining can be used appropriately.

A target of the fluorescent dye staining is not particularly limited, but is preferably a substance that is specifically present in a vascular endothelial cell, and is preferably a sugar chain that is specifically expressed in the vascular endothelial cell.

<Dye staining>

In the imaging step (2), dye staining with a dye for bright field observation and bright field imaging may be further performed. The dye staining may be performed for observing the form of a cell or the like contained in a sample, or may be performed for observing a substance that is specifically present in a vascular endothelial cell, and may be a sugar chain that is specifically expressed in the vascular endothelial cell.

<Drug evaluation method>

As described above, the drug evaluation method of the present invention performs the drug administration step (A); the imaging step (1) a plurality of times or the imaging step (2); and further evaluates a drug involved in angiogenesis using an image acquired in the imaging step (1) or (2). The drug evaluation method preferably evaluates the drug by acquiring information including blood vessel information described later from the image.

Specifically, for example, in a case where the imaging step (1) is performed, an image representing a blood vessel of a subject is obtained by metal nanoparticles present in the blood vessel by a radiography device, and the drug can be evaluated using the image, preferably by acquiring blood vessel information described later from the image.

For example, also in a case where the imaging step (2) is performed, drug evaluation is performed using an acquired fluorescence image. In this case, the drug evaluation can be performed based on fluorescence emission representing the target protein. Specifically, in a case where the dimeric protein is VEGF or PDGF, drug evaluation can be performed based on fluorescence emission representing VEGFR or PDGFR to be specifically bonded to VEGF or PDGF. Blood vessel information described later can be acquired preferably from the fluorescence emission.

As information other than blood vessel information in the information including the blood vessel information, pathological information is preferably included. In evaluating a drug, the drug may be evaluated by acquiring information other than the information including blood vessel information (hereinafter, referred to as "other information").

A drug can be evaluated by, for example, setting a certain threshold for arbitrary information and classifying a test control group. For example, regarding a change ratio of a blood vessel volume, 50% or more is judged to be highly effective (Rank A), 30% or more and 50% or less is judged to be moderately effective (Rank B), and the other cases are judged to be poorly effective (Rank C). By setting a threshold for a plurality of pieces of information and making a composite judgment, more accurate drug evaluation can be performed.

<Blood vessel information>

The blood vessel information is information on a blood vessel of a subject, and preferably includes information on the volume of a blood vessel in a lesion, and more preferably further includes positional information of the blood vessel in the lesion. Furthermore, information on the distribution of blood vessels in a lesion, a change in the volume of the blood vessels in the lesion before and after drug administration, a change in the distribution of blood vessels in the lesion before and after drug administration, and the like may be included.

The blood vessel information may be acquired from an image acquired by setting a lesion and surroundings thereof as a region of interest (ROI), or may be acquired from an image acquired by setting the entire subject as a region of interest.

For example, in a case where a localization pattern of blood vessels is acquired, a distribution state can be further classified into some arbitrary patterns (for example, accumulation at the center of a lesion, distribution at a periphery of the lesion, uneven distribution over the entire lesion, and the like).

<Pathological information>

The pathological information preferably includes the volume of a lesion, and more preferably includes positional information of the lesion. Furthermore, a change in the volume of the lesion before and after drug administration may be included.

<Other information>

In the information acquiring step, the other information other than the blood vessel information and the pathological information can also be acquired. Such information and a method for acquiring the information are not particularly limited. However, examples thereof include a protein expression level that can be detected by a means such as immunostaining using a fluorescent dye-bonded antibody, a sugar uptake amount that can be measured by a means such as FDG-PET test (cellular glucose metabolism level), and a blood flow velocity and a blood flow velocity distribution that can be measured by a means such as MRI or ultrasonic waves. The other information may include information obtained by combining a plurality of pieces of information such as information including blood vessel information and pathological information. For example, by determining a value obtained by dividing a blood vessel volume by the volume of a lesion ((volume of blood vessel)/(volume of lesion)), the occupancy of a blood vessel in the lesion and a change thereof can be determined.

<Dimeric protein>

The dimeric protein of the present invention contains a first monomer and a second monomer, and is used for detecting a target protein by being specifically bonded to the target protein expressed on a cell surface.

The dimeric protein contains a first monomer which is a fusion peptide fused to a tag bonded to streptavidin at one end, and a second monomer. The second monomer is preferably fused to a tag bonded to a protein other than streptavidin at one end. In other words, the dimeric protein of the present invention preferably contains a first monomer which is a fusion peptide fused to a tag bonded to streptavidin at one end, and a second monomer fused to a tag bonded to a protein other than streptavidin at one end.

(First monomer)

The first monomer is a peptide that becomes the dimeric protein of the present invention by being bonded to a second monomer described later, and is a fusion peptide fused to a tag bonded to streptavidin only at one end. The peptide is not particularly limited as long as being able to constitute the dimeric protein, but is preferably a linear peptide, in which only either a C-end or an N-end of the peptide is fused to a tag bonded to streptavidin.

As described above, preferably, the first monomer has only one tag bonded to streptavidin in a structure thereof, and has no tag bonded to a protein other than streptavidin.

(Second monomer)

The second monomer of the present invention is a peptide that becomes the dimeric protein of the present invention by being bonded to the first monomer, and is usually a fusion peptide fused to a tag bonded to a protein other than streptavidin only at one end. The tag fused to an end of the second monomer is not particularly limited, but a tag that does not interact with streptavidin is preferably selected. The peptide is not particularly limited as long as being able to constitute the dimeric protein, but is preferably a linear peptide, in which only either a C-end or an N-end of the peptide is fused to a tag bonded to a protein other than streptavidin.

As described above, preferably, the second monomer has only one tag bonded to a protein other than streptavidin in a structure thereof, and has no tag bonded to streptavidin.

Therefore, the dimeric protein of the present invention has only one tag that can be bonded to streptavidin in one molecule, and preferably further has only one tag bonded to a protein other than streptavidin.

A bonding mode between the first monomer and the second monomer is not particularly limited, and the first monomer and the second can be bonded to each other through, for example, a covalent bond such as a disulfide bond, an ionic bond, or a hydrogen bond. The first monomer and the second monomer are preferably linear as described above, and both the first monomer and the second monomer are particularly preferably linear. The peptide sequences of the first monomer and the second monomer may be the same as each other except for a tag, or may be different from each other. Which of the two peptides contained in the dimer is the first monomer and which of the two peptides contained in the dimer is the second monomer can be arbitrarily determined according to the properties and structure of the dimeric protein.

The dimeric protein of the present invention is not particularly limited, but is preferably a protein derived from a protein to be specifically bonded to a target protein expressed on a cell surface.

For example, in a case where the target protein is a vascular endothelial cell growth factor receptor (VEGFR) or a platelet-derived growth factor receptor (PDGFR), the dimeric protein is preferably derived from a vascular endothelial cell growth factor (VEGF) or a platelet-derived growth factor (PDGF) to be specifically bonded to VEGFR or PDGFR.

Specifically, when the target protein is VEGFR, and the dimeric protein is derived from VEGF, the first monomer is a peptide of a sequence fused to a tag bonded to streptavidin only at one of the ends of a monomer peptide constituting VEGF, and the second monomer is preferably a peptide of a sequence fused to a tag bonded to a protein other than streptavidin only at one of the ends of the monomer peptide constituting VEGF. In other words, in this case, in the dimeric protein of the present invention, a tag bonded to streptavidin is fused only to one end of VEGF, and preferably a tag bonded to a protein other than streptavidin is fused only to one end of a monomer not fused to the tag bonded to streptavidin.

<Fluorescent labeling and staining method>

A fluorescent labeling and staining method according to an embodiment of the present invention is a method for staining a target protein by bonding the fluorescent label to the target protein.

The fluorescence staining method may be a method for staining a target protein by bonding the target protein to a dimeric protein and further bonding the bonded protein to the fluorescent label, or a method for staining the target protein by bonding the target protein to a fluorescence labelling probe (described later) in which a fluorescent label is bonded to the dimeric protein in advance.

As the former form, specifically, by bringing the dimeric protein in contact with a tissue section or a cell collected from a subject that is an animal having a lesion, a target protein that can be present in the tissue section or the like is bonded to the dimeric protein. Furthermore, by bringing the tissue section or the like in contact with the dimeric protein in contact with the fluorescent label to bond the fluorescent label to the dimeric protein in the tissue section, the target protein is fluorescently stained.

As the latter form, specifically, by bonding the tissue section or cell to the fluorescence labelling probe, the target protein can be stained.

As described above, the dimeric protein of the present invention has only one tag bonded to streptavidin per molecule, and the fluorescent label is a fluorescent dye or the like bonded to streptavidin. Therefore, one molecule of the fluorescent label is bonded to one molecule of the dimeric protein. Therefore, specifically, by bonding streptavidin which is a component of the fluorescent label to a "tag bonded to streptavidin" fused to an end of the first monomer constituting the dimeric protein specifically bonded to the target protein, staining is performed.

<Fluorescent dye staining>

In the fluorescent labeling and staining method, fluorescent dye staining performed using something other than a fluorescence labelling probe or a fluorescence staining liquid described later may also be performed. The fluorescent dye staining is preferably fluorescence staining performed using a fluorescent dye, a quantum dot, a phosphor-integrated nanoparticle, or the like as described above. A target of the fluorescent dye staining is not particularly limited, but is preferably a substance that is specifically present in a vascular endothelial cell, and is preferably a sugar chain that is specifically expressed in the vascular endothelial cell.

In the staining method, dye staining with a dye for bright field observation and bright field imaging may also be performed. The dye staining may be performed for observing the form of a cell or the like contained in a sample, or may be performed for observing a substance that is specifically present in a vascular endothelial cell, and may be a sugar chain that is specifically expressed in the vascular endothelial cell.

<Fluorescence labelling probe>

An embodiment of the present invention provides a fluorescence labelling probe which is a complex obtained by bonding the fluorescent label in advance to the dimeric protein before fluorescence staining. Specifically, by bonding streptavidin which is a component of the fluorescent label to a "tag bonded to streptavidin" fused to an end of the first monomer constituting the dimeric protein, the fluorescence labelling probe of the present invention is constituted. As described above, the dimeric protein of the present invention has only one tag that can be bonded to streptavidin per molecule. Therefore, the fluorescence labelling probe of the present invention has a configuration in which only one molecule of a fluorescent label is bonded to one molecule of the dimeric protein.

<Fluorescence staining liquid>

A fluorescence staining liquid according to an embodiment of the present invention contains the fluorescence labelling probe. In general, the fluorescence staining liquid can be prepared as a dispersion obtained by preparing and collecting a fluorescence labelling probe, and then dispersing the fluorescence labelling probe in an appropriate dispersion medium, for example, PBS (phosphate-buffered saline) containing 1% BSA.

In a case where the fluorescence staining liquid is used in an embodiment in which two or more types of target proteins are used as a target of fluorescent labeling, two or more types of fluorescence labelling probes corresponding to the target proteins, respectively, may be contained. In this case, peaks of fluorescence wavelengths are preferably separated from each other sufficiently, and are preferably separated from each other by, for example, 100 nm or more such that the two or more types of fluorescence labelling probes do not adversely affect discrimination of fluorescence (bright spot) of the fluorescence labelling probes that label the target proteins. The fluorescence staining liquid using such a plurality of target proteins as a target may be a one-pack type in which two or more types of fluorescence labelling probes are contained in the same pack (dispersion), or may be a multi-pack type in which fluorescence premix particles are contained in separate packs. Depending on an embodiment of the staining method, the fluorescence staining liquid may include, in addition to a one-pack or a multi-pack of fluorescence labelling probes, a pack of another reagent (for example, a staining liquid for dye staining described later).

<Method for producing dimeric protein>

A method for producing the dimeric protein of the present invention includes: a purification step of mixing the first monomer and the second monomer; and purifying a dimeric protein containing the first monomer and the second monomer by removing contaminants from the obtained mixture.

A method for producing the first monomer and the second monomer is not particularly limited, and a peptide artificially synthesized by a known method may be used as each of the monomers, or a peptide expressed by introducing a gene into E. coli may be used as each of the monomers.

The dimeric protein of the present invention is produced by mixing the first monomer and the second monomer in an appropriate environment. At this time, the reaction solution after mixing includes, in addition to the dimeric protein of the present invention, the first monomer that has not reacted, the second monomer that has not reacted, a dimer containing only the first monomer, and a dimer containing only the second monomer as contaminants.

The producing method of the present invention includes a purification step of removing these contaminants from the mixed solution, and the purification step is preferably performed using a column, and is more preferably performed stepwise using two types of purification columns. Preferably, by sequentially causing the mixed reaction solution to pass through a first purification column carrying a carrier to be specifically bonded to a tag fused to the first monomer, and a second purification column carrying a carrier to be specifically bonded to a tag fused to the second monomer, purification can be performed. At this time, a step of causing the mixed reaction solution to pass through the first purification column is referred to as a first purification step, and a step of causing the mixed reaction solution to pass through the second purification column is referred to as a second purification step. Either the first purification step or the second purification step may be performed first.

By removing the second monomer that has not reacted and the dimer containing only the second monomer in the first purification step, and further removing the first monomer that has not reacted and the dimer containing only the first monomer in the second purification step, the dimeric protein of the present invention is purified.

<First purification column and second purification column>

The first and second purification columns of the present invention (hereinafter, also collectively referred to as purification columns) each include a carrier having a pore with a size that allows a dimeric protein to be purified to pass. The carrier carried on the first purification column preferably has a property of being specifically bonded to a tag fused to the first monomer, and the carrier carried on the second purification column preferably has a property of being specifically bonded to a tag fused to the second monomer. Specifically, in a case where strep-tag is used as a tag, a commercially available resin for fixing a streptavidin analog may be used, or one in which a streptavidin analog is fixed to an arbitrary matrix may be produced. In a case where His-tag (registered trademark) is used as a tag, a commercially available carrier such as nickel-nitrilotriacetic acid (Ni-NTA) may be used, or a metal ion to be bonded to His-tag, such as nickel, or one in which an antibody specific to His-tag is bonded to a matrix may be produced.

The matrix of such a carrier is not particularly limited, and matrices of various materials such as a porous polymer formed of a crosslinked copolymer can be used. For example, a porous polymer formed of a crosslinked copolymer of dextran or a derivative thereof (such as allyl dextran) and acrylamide or a derivative thereof (such as N,N-methylenebisacrylamide) is preferably used.

The "producing method" of the present invention usually includes, after each purification step, an elimination step of eliminating a peptide captured by a carrier from the carrier. This elimination step can be performed, for example, by causing an elimination liquid to pass through the carrier.

The elimination liquid is not particularly limited, but is preferably a liquid that can sufficiently eliminate the dimeric protein from the carrier, and furthermore, more preferably a liquid that does not cause the structure or function of the dimeric protein to disappear. For example, glycine-HCl is preferably used.

<Physiological activity evaluation method>

A physiological activity evaluation method according to an embodiment of the present invention is a method for evaluating the physiological activity of a target protein using the dimeric protein. A means used for the evaluation is not particularly limited. However, examples thereof include a method for evaluating the physiological activity of a target protein included in a cell by administering the dimeric protein to the cell and then observing movement of the cell or a biological substance included in the cell.

(Acquisition of image)

As described above, the drug evaluation method of the present invention is a method for evaluating a drug involved in angiogenesis using an image acquired in the imaging step (1) or (2), and preferably evaluates the drug by acquiring information including blood vessel information from the image. Therefore, the drug evaluation method of the present invention includes a step of acquiring an image acquired in the imaging step (1) or (2). The image is preferably a digital image, and is more preferably acquired as stereoscopic image information.

For use in drug evaluation in the drug evaluation method of the present invention, the image may be analyzed based on an arbitrary algorithm (image analysis or the like), or the image may be converted quantitatively by any method, such as numerical conversion or graphing. Such analysis and conversion can be performed, for example, using a commercially available image analysis system.

<Subject>

A subject in the present invention is a human or a non-human animal having a lesion, preferably an experimental animal having a lesion. The lesion is not particularly limited, but is preferably a tumor part. Specific examples thereof include a solid cancer such as cytoma, melanoma, sarcoma, brain tumor, head and neck cancer, stomach cancer, lung cancer, breast cancer, liver cancer, colorectal cancer, cervical cancer, prostate cancer, or bladder cancer, leukemia, lymphoma, and multiple myeloma.

The experimental animal is preferably selected depending on a purpose, and a disease model animal is particularly preferably used. For example, when the lesion is a tumor part, as the disease model animal, for example, a tumor-bearing animal previously holding a tumor part derived from a tumor cell or tumor tissue and generated in vivo is preferably used.

In a case where a tumor-bearing animal model is used as the experimental animal, a method for causing the experimental animal to hold a tumor part is not particularly limited, and a known method can be used.

Examples of animal species used as the experimental animal include an animal genetically controlled to a certain degree and having homogeneous genetic requirements, such as a mouse, a rat, a rabbit, a guinea pig, a gerbil, a hamster, a ferret, a dog, a miniature pig, a monkey, a cow, a horse, or a sheep. Particularly, a mouse is widely used.

As the tumor-bearing model mouse, cancer-bearing mice can be broadly classified into three types: chemically expressed model mice, genetically modified model mice, and xenograft model mice (see the table below; Kohrt et al., Defining the optimal murine models to investigate immune checkpoint blockers and their combination with other immunotherapies. Annals of Oncology (2016) 27 (7): 1190-1198).

The xenograft model mouse is produced by transplanting a cultured cell derived from a tumor cell taken from a patient into an immunodeficient mouse. Examples of a mouse into which a human-derived cultured cancer cell has been transplanted include a cell-line derived xenograft (CDX) model mouse. Examples of a mouse into which a tumor tissue derived from a human (patient) has been transplanted include a patient derived xenograft (PDX) model mouse, an immuno-avatar model mouse, a hemato-lymphoid humanized model mouse, and an immune-PDX model mouse.

The PDX mouse is produced by transplanting a tumor tissue derived from a patient into an immunodeficient mouse. The immuno-avatar model mouse, the hemato-lymphoid humanized model mouse, and the immune-PDX model mouse are produced by transplanting a tumor tissue derived from a patient into an immunodeficient mouse (immunized humanized mouse) into which a human peripheral blood mononuclear cell, a CD34 + human hematopoietic stem cell and a precursor cell (HSPC) thereof, and a tumor-infiltrating lymphocyte have been transplanted, respectively.

[Table A]

| | Cancer cell | Immune cell | Model |
|---|---|---|---|
| Chemically expressed model mouse | Mouse | Mouse | Model in which a carcinogenic substance is administered to generate cancer |
| Genetically modified model mouse | Mouse | Mouse | Model in which a gene is mutated to generate cancer |
| Cultured cancer cell transplantation mouse (CDX model mouse) | Human | None | Model in which a human tumor-derived cultured cancer cell is transplanted into an immunodeficient mouse to generate cancer |
| Patient-derived cancer cell transplantation mouse (PDX model mouse) | Human | None | Model in which a cancer cell collected from a specific patient is transplanted into an immunodeficient mouse to generate cancer |
| Cultured cancer cell transplantation immunized humanized mouse (immuno-avatar model mouse and hemato-lymphoid humanized model mouse) | Human | Human | Model in which a human tumor-derived cultured cancer cell is transplanted into an immunized humanized mouse to generate cancer |
| Patient tumor tissue transplantation immunized humanized mouse (immune-PDX model mouse) | Human | Human | Model in which a cancer cell collected from a specific patient is transplanted into an immunized humanized mouse to generate cancer |

In the drug evaluation method of the present invention, specifically, for example, in the imaging step (2), when a plurality of PDX mice obtained by transplanting tumor tissues derived from the same patient into syngeneic mice is produced, an effect of a drug can be evaluated by measuring the size of a tumor and a change thereof at one or more time points before and after administration of the drug.

<Evaluation system>

A drug evaluation system according to an embodiment of the present invention is a system for performing the drug evaluation method, and a drug evaluation system including an imaging device including at least one of a radiography device and a fluorescence imaging device, and an information processing device.

The radiography device is a device that performs radiography. The fluorescence imaging device is a device that performs fluorescence imaging. In a case where the imaging device includes both the radiography device and the fluorescence imaging device, radiography and fluorescence imaging may be performed at the same time, or imaging corresponding to either one of the devices may be performed using the device. The imaging device may further include a device for performing bright field imaging.

The information processing device evaluates a drug involved in angiogenesis using an image received from an image acquired by the radiography device or the fluorescence imaging device, and preferably evaluates a drug involved in angiogenesis further using information including blood vessel information acquired from the received image. The information processing device may further include a display device or an information storage device.

(Radiography device)

The radiography device is not particularly limited as long as being able to perform radiography on a subject, but is preferably a three-dimensional radiography device. Usually, the radiography device includes a radiation source that emits radiation and a radiation image detector that detects the radiation, and performs imaging by emitting the radiation while rotating an irradiation unit and the detector that rotate around a support table on which a subject is fixed, and moving the support table based on an instruction from an input device.

Examples of the radiation source include a Coolidge X-ray tube and a rotating anode X-ray tube widely used in a medical site.

The radiation detector usually includes a plurality of detection elements, detects radiation emitted from the radiation source and radiation that has passed through a subject, and outputs an electric signal corresponding to the intensity of the radiation. The radiation detectors are roughly classified into two types, a scintillation detector and a semiconductor detector. In the scintillation detector, radiation is converted into light by a scintillator, and the light is further converted into an electric signal by a photodiode. In the semiconductor detector, radiation is directly converted into an electric signal. The electric signal thus converted is transmitted as an image to the information processing device by digital radiography.

(Fluorescence imaging device)

The fluorescence imaging device is not particularly limited as long as being able to capture a fluorescence image, but is preferably a fluorescence microscope including a fluorescence imaging instrument, and more preferably a confocal microscope including a fluorescence imaging instrument. The fluorescence imaging instrument is not particularly limited as long as being able to capture a fluorescence image, and examples thereof include a digital camera attached to the fluorescent microscope or the confocal microscope. A fluorescence image acquired by the fluorescence imaging device is transmitted to the information processing device.

(Device for performing bright field imaging)

The device for performing bright field imaging is not particularly limited as long as being able to observe a cell or a substance as a target of dye staining in a tissue section stained with a dye for the bright field observation. An optical microscope or a substance microscope including an optical imaging instrument is typically used. In a case where the fluorescence imaging device is a fluorescence microscope or a confocal microscope including a fluorescence imaging instrument, and the microscope has a function as an optical microscope or is integrated with the optical microscope, the fluorescence imaging device can also be used as the device for performing bright field imaging.

The fluorescence imaging instrument is not particularly limited as long as being able to capture a bright field image, and examples thereof include a digital camera attached to the fluorescent microscope or the confocal microscope. An image acquired by the device for performing bright field imaging is transmitted to the information processing device.

(Information processing device)

As described above, the information processing device included in the evaluation system of the present invention evaluates a drug involved in angiogenesis using an image received from an image acquired by the radiography device or the fluorescence imaging device. Specifically, the information processing device analyzes an image received from the imaging device, and evaluates a drug involved in angiogenesis using the obtained analysis result. An analysis method is not particularly limited, but the image may be directly analyzed based on any algorithm (for example, a nearest neighbor method, a bilinear interpolation method, a bicubic interpolation method, or a Lanczos interpolation method) (image analysis or the like), or the image may be converted quantitatively by any method, such as numerical conversion or graphing, for analysis.

The information processing device preferably evaluates a drug involved in angiogenesis further using information including blood vessel information acquired from an image received from the radiography device or the fluorescence imaging device.

The evaluation system of the present invention preferably further includes an input device that controls the system and a display device that outputs information acquired by the information processing device.

The information processing device preferably includes an information storage unit that stores information including blood vessel information. For example, at a time point of measurement, by inputting ID information of a subject and ID information of a drug from the input device or the like, performing imaging at a plurality of time points, and storing data in the information processing device, information including blood vessel information for a specific individual can be referred to over time. In addition, by performing a measurement over time as described above, the amount of change in a measured value, a ratio thereof, a fluctuation thereof, and the like can be observed.

The measurement of each value desirably includes a time point before drug administration, but the amount of change may be calculated from a plurality of measurements only after administration.

<Display device>

The evaluation system preferably further includes a display device that displays at least one of the information including blood vessel information, the analysis result, and drug evaluation.

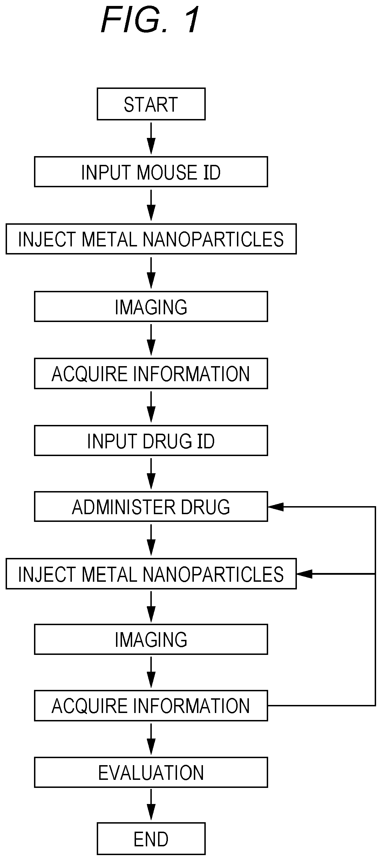

The display device includes, for example, a monitor such as a cathode ray tube (CRT) or a liquid crystal display (LCD), and displays information acquired by the information processing device according to display control of the input device. The display device can preferably display an image (image information) in addition to the information including blood vessel information. Information to be displayed may be information that is further arranged by any method. For example, drug ID (drug name, LOT number, or the like), subject information (mouse ID, patient information, or the like), drug administration and imaging history, each information change amount, measurement date and time, captured image, and drug judgement result in each subject are also preferably displayed (see Fig. 1: display device).

<Information storage device>

The evaluation system preferably includes an information storage unit that stores display device information that displays at least one of the image, the information including blood vessel information, the analysis result, and drug evaluation. For example, at a time point of measurement, by inputting ID information of a sample and ID information of a drug from the input device or the like, performing imaging at a plurality of time points, and storing data in the information processing device, an image obtained over time, a change in blood vessel information, or the like for a certain sample can be referred to.

<Program>

A program according to an embodiment of the present invention is a program for causing a computer to execute the drug evaluation method. For example, the program is a program for causing a computer to execute, in the evaluation system, a process of capturing an image with a radiography device or a fluorescence imaging device, a process of presenting the image to an information processing device, a process of detecting information including blood vessel information and other information from an image received by the information processing device, a process of analyzing the image or information, a process of calculating changes over time in images or pieces of information acquired at a plurality of time points, a process of evaluating a drug administered to a subject based on results of the above processes, a process of displaying the drug evaluation on a display device, and transmitting the drug evaluation to an information storage device, and the like.

The program may be stored in an information processing device, may be recorded in another computer-readable recording medium such as a magnetic tape (a digital data storage (DSS) or the like), a magnetic disk (a hard disk drive (HDD), a flexible disk (FD), or the like), an optical disc (a compact disc (CD), a digital versatile disc (DVD), a Blu-ray disc (BD), or the like), a magneto-optical disk (MO), a flash memory (solid state drive (SSD)), a memory card, or a USB memory, or may be provided independently. In a case where the program is stored in an information processing device or a readable medium, the information processing device or the like preferably stores also data necessary for executing the program. Examples of the data include subject information such as a patient ID or a patient name, drug information such as a drug ID, and imaging site (subject site) information.

Examples

Hereinafter, a preferable embodiment of the present invention will be described more specifically based on Examples, but the present invention is not limited to these Examples.

[Production Example 1]

<Production of cancer-bearing mouse>

$5 \times 10^6$ human ovarian cancer-derived cultured cell lines SKOV3 (obtained from American type culture collection (ATCC)) were transplanted subcutaneously into the back of a sevefe combined immunodeficiency (SCID; Charles River Co.; BALB/c-nu)) mouse to produce a cancer-bearing mouse.

[Production Example 2]

<Production of PEG-modified gold nanoparticles>

In order to prepare PEG-modified gold nanoparticles, 99.4 mg of chloroauric acid (Strem Chemicals Inc., Newburyport, MA, USA) was dissolved in 233 ml of ultrapure water and heated to boiling. The chloroauric acid solution was stirred vigorously, and 28 ml of 39 mM sodium citrate was added to the solution with constant stirring. The sample was boiled for 30 minutes.

The solution was cooled to 25°C. Thereafter, 99.4 mg of poly (ethylene glycol) 2-mercaptoethyl ether acetic acid (HS-PEG-COOH, NANOCS Inc., product name Thiol PEG Acid, model number PG2-CATH-3k) was added thereto, and the resulting solution was incubated at 25°C for 12 hours with stirring. After the incubation, the solution was washed by repeating centrifugation at 22000 g for 40 minutes and suspension in PBS three times. Finally, the solution was resuspended in PBS, and PEG-modified gold nanoparticles having a particle diameter of 15 nm were thereby prepared so as to have a final concentration of 0.6 M.

[Example 1]

<Drug evaluation using gold nanoparticles>

Bevacizumab (Avastin (registered trademark), Chugai Pharmaceutical Co., Ltd.), which is an antibody drug used as an anticancer agent, was evaluated using the cancer-bearing mouse produced in Production Example 1 and the PEG-modified gold nanoparticles produced in Production Example 2.

Three weeks and five weeks after cancer cell transplantation, to the cancer-bearing mouse, a solution of bevacizumab (Avastin (registered trademark), Chugai Pharmaceutical Co., Ltd.) prepared so as to have a concentration of 25 $\mu$g/$\mu$L with saline was administered from the tail vein at a concentration of 5 $\mu$g/g in terms of bevacizumab. The same amount of saline was administered to a cancer-bearing mouse similarly produced as a control.

A mouse body weight and a tumor volume were measured at three, five, and seven weeks after the cancer cell transplantation. A short axis (a) and a long axis (b) of a tumor were measured using a caliper, and the volume of the tumor was determined by the following formula.

$$\text{“Volume”} = (4/3)\,\pi a^{\wedge}2 \times b$$

At the same time points as measurement of the body weight, 200 $\mu$L of a 0.6 M PEG-modified gold nanoparticle solution produced in Production Example 2 was administered from the tail vein. Immediately after administration, X-ray CT imaging was performed at a pixel size of 9 microns, 50 kVp, and 490 $\mu$A using an X-ray micro CT scanner SkySan 1176 for small animals (Bruker). CT signals were quantified in Hounsfield units (HU).

At each time point, a captured image was observed, and the volume of a tumor, the density and volume of blood vessels, and a ratio of the blood vessel volume to the tumor volume (blood vessel volume/cancer volume) were acquired using image analysis software CTVox (Bruker).

When a result three weeks after cancer cell transplantation is compared with a result five weeks after the transplantation, the tumor volume five weeks after the transplantation became 4.8 times the tumor volume three weeks after the transplantation in the saline administration group, and the tumor volume five weeks after the transplantation became 3.8 times the tumor volume three weeks after the transplantation in the bevacizumab administration group. A large difference was not observed in the results. Meanwhile, a result five weeks after the transplantation is compared with a result seven weeks after the transplantation, the tumor volume seven weeks after the transplantation became 4.0 times the tumor volume five weeks after the transplantation (the tumor volume seven weeks after the transplantation became 19.2 times the tumor volume three weeks after the transplantation) in the saline administration group, and the tumor volume seven weeks after the transplantation became 2.0 times the tumor volume five weeks after the transplantation (the tumor volume seven weeks after the transplantation became 7.6 times the tumor volume three weeks after the transplantation) in the bevacizumab administration group. This indicates that an increase in tumor volume was significantly suppressed in the bevacizumab administration group.

Similarly in the blood vessel volume, when a result three weeks after the transplantation is compared with a result five weeks after the transplantation, the blood vessel volume five weeks after the transplantation became 5.0 times the blood vessel volume three weeks after the transplantation in the bevacizumab administration group, and the blood vessel volume five weeks after the transplantation became 6.4 times the blood vessel volume three weeks after the transplantation in the saline administration group. A large difference was not observed in the results. However, a result five weeks after cancer the is compared with a result seven weeks after the transplantation, the blood vessel volume seven weeks after the transplantation became 7.8 times the blood vessel volume five weeks after the transplantation (the blood vessel volume seven weeks after the transplantation became 49.9 times the blood vessel volume three weeks after the transplantation) in the saline administration group, and the blood vessel volume seven weeks after the transplantation became 2.8 times the blood vessel volume five weeks after the transplantation (the blood vessel volume seven weeks after the transplantation became 14.0 times the blood vessel volume three weeks after the transplantation) in the bevacizumab administration group. This indicates that an increase in blood vessel volume was significantly suppressed in the bevacizumab administration group.

Similarly in the ratio of a blood vessel volume, when a result three weeks after the transplantation is compared with a result five weeks after the transplantation, the ratio five weeks after the transplantation became 1.4 times the ratio three weeks after the transplantation in the saline administration group, and the ratio five weeks after the transplantation became 1.4 times the ratio three weeks after the transplantation in the bevacizumab administration group. A difference was not observed in the results. However, a result five weeks after the transplantation is compared with a result seven weeks after the transplantation, the ratio seven weeks after the transplantation became 1.9 times the ratio five weeks after the transplantation (the ratio seven weeks after the transplantation became 2.7 times the ratio three weeks after the transplantation) in the saline administration group, and the ratio seven weeks after the transplantation became 1.1 times the ratio five weeks after the transplantation (the ratio seven weeks after the transplantation became 1.6 times the ratio three weeks after the transplantation) in the bevacizumab administration group. This indicates that an increase in blood vessel volume was significantly suppressed in the bevacizumab administration group.

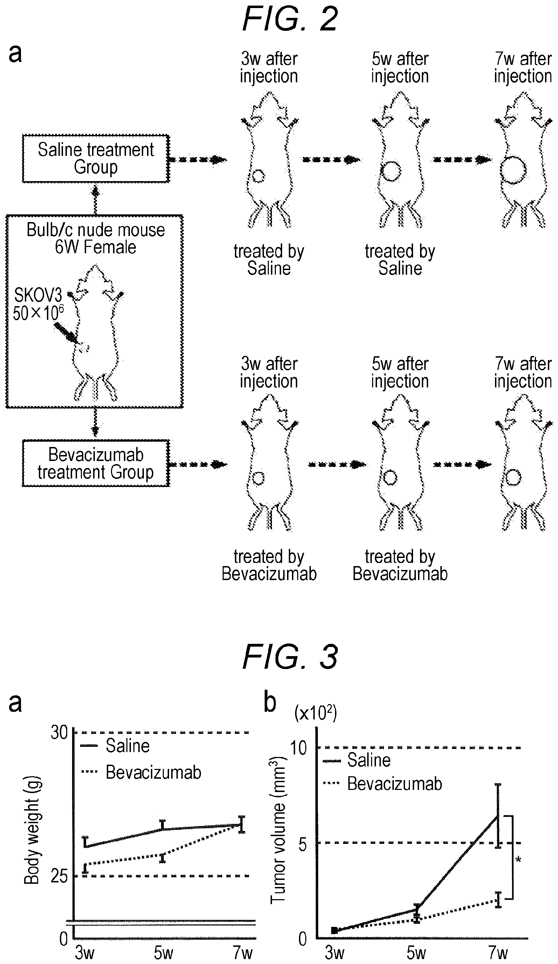

Fig. 3 illustrates the above results. Three to five weeks after the cancer cell transplantation, there was no large difference in the tumor volume, the blood vessel volume, and the ratio of the blood vessel volume between a mouse to which bevacizumab had been administered and a mouse to which saline had been administered. Meanwhile, five to seven weeks after the cancer cell transplantation, a mouse to which bevacizumab had been administered suppressed an increase in the tumor volume, the blood vessel volume, and the ratio of the blood vessel volume more significantly than a mouse to which saline had been administered. Therefore, it has been found that bevacizumab also has an anti-tumor effect and an anti-angiogenic effect, and that at least two or more administrations of the drug would be required to expect the effects.

[Comparative Example 1]

X-ray CT imaging was performed on a mouse to which bevacizumab had been administered and a mouse to which saline had been administered in a similar manner to Example 1 except that no gold nanoparticles were administered.

The volume of a tumor part was determined, and the anti-tumor effect could be confirmed. However, a blood vessel could not be observed, and the anti-angiogenic effect could not be confirmed.

(Discussion)

In the above experiment, as can be seen from the graphs illustrated in k) to m) of Fig. 4, seven weeks after the cancer cell transplantation administration, no significant difference was observed in any one of the tumor volume, the blood vessel volume, and the ratio of the blood vessel volume to the tumor volume between the experimental group and the control group. Meanwhile, seven weeks after the cancer cell transplantation, that is, two weeks after two administrations of bevacizumab, it was found that there was a large difference in any one of the tumor volume, the blood vessel volume, and the ratio of the blood vessel volume to the tumor volume between the experimental group and the control group. As described above, it is found that the efficacy of an angiogenesis inhibitor can be evaluated by visualizing a blood vessel and a tumor over time using a living mouse, numerically converting a tumor volume and a blood vessel volume, and observing changes thereof. Furthermore, since it is not necessary to sacrifice a subject such as a mouse in drug evaluation by this method, it is considered that an advantage can be obtained from a viewpoint of animal protection that an experimental animal is not wasted.

In addition, the above results suggest that a single administration of bevacizumab is not sufficient for the effect of the drug, and that a plurality of administrations is important. Therefore, it is possible to present a possibility that a method for administering a drug, a preferable combination of drugs, and the like can be determined by performing such an evaluation.

In addition, by evaluating the blood vessel volume and the ratio of the blood vessel volume to the tumor volume with high accuracy, it is possible to evaluate whether an anticancer effect of the drug is based on an angiogenesis inhibitory effect, and the evaluation may be used for screening of a new candidate drug. Furthermore, since it is not necessary to sacrifice a subject such as a mouse, it is considered that an advantage can be obtained from a viewpoint of animal protection that an experimental animal is not wasted.

[Production Example 3]

<Strep-tag (registered trademark)-fused VEGF subunit>

• Production of cDNA

A mixed liquid containing 20 ng of purified Mouse Vegf164 cDNA (GE Dharmacon), 10 μM 5'-primer (5'-ATACCATGGCACCCACGACA-3'), 10 μM 3'-primer (5'-TGTTCGGTTCCGCCGAGCTCAAA-3'), and KOD Fx (Toyobo Co., Ltd.) was put in a PCR device (Takara PCR Thermal Cycler Dice (registered trademark)) Gradient; (Takara Bio Inc.) according to a program of 98°C for two minutes → 30 × (98°C for 10 seconds → 55°C for 30 seconds → 72°C for 90 seconds) to amplify DNA.

The obtained PCR product was migrated on a 1.5% (w/v) agarose gel in a 1 × TAE buffer, and stained with SYBER Green (Takara Bio Inc.) to extract a target band. Purification was performed using Wizard (registered trademark) SV GEL and PCR Clean Up system (both available from Promega Corporation).

The purified PCR product and Strep-tag (registered trademark) expression vector pASK-IBA63a-plus (IBA) were treated with restriction enzymes Ncol and Xhol, and then mixed at a molar ratio of 2:1. The mixed liquid was further mixed with the same amount of DNA Ligation Kit <Mighty Mix> (product code 6023, Takara Bio Inc.). The resulting liquid was incubated at 16°C for 12 hours to perform ligation. The obtained reaction solution was subjected to migration, staining, extraction, and purification in a similar manner to the PCR product described above.

E. coli competent cells DH5α (Toyobo Co., Ltd.) were transformed using the purified reaction solution, and a colony was selected using an LB plate containing 200 μg/mL ampicillin. Regarding both cDNAs, from a colony selected using QIAprep Spin Miniprep Kit (Qiagen), Strep-tag-fused VEGF164 cDNA was purified.

As a result of entrusting the DNA sequence of the purified cDNA to Eurofin Genomics and analyzing the DNA sequence, it was confirmed that a Strep-tag was bonded to a C-end of VEGF164 in the sequence.

• Expression of protein

Using the purified cDNA, BL21 Rosetta (registered trademark) 2 (DE3) competent cells (Merck Millipore) were transformed. A colony was selected using an LB plate containing 200 μg/mL ampicillin and 20 μg/mL chloramphenicol. A mixed liquid containing the selected colony, 10 μM 3'-primer (5'-TGACGCAGTAGCGGTAAACGGCAGAC-3'), 10 μM 5'-primer (5'-GAGTTATTTTACCACTCCCTATCAGTG-3'), and Go-Taq (registered trademark) Green Master Mix (Promega Corporation) was subjected to colony PCR according to a program similar to that described above, and was subjected to electrophoresis to confirm that Strep-tag-fused VEGF164 cDNA had been introduced into the selected colony.

The selected colony was cultured in 25 ml of an LB medium containing 200 μg/mL ampicillin and 20 μg/mL chloramphenicol at 37°C at 160 rpm for 12 hours, then transferred to 200 mL of an LB medium having the same composition, incubated at 37°C at 160 rpm for 60 minutes. Furthermore, 50 μg of anhydrotetracycline was added to the medium, and the resulting mixture was further incubated at 37°C at 160 rpm for six hours to induce expression of Strep-tag-fused VEGF164. Thereafter, centrifugation was performed at 4°C at 6000 g for 15 minutes, and precipitated E. coli was collected.

The collected pellet was mixed well with 2 mL of PBS including 2 μL of DNase (Promega Corporation), 2 μL of Benzonaze (Sigma-Aldrich), and 20 μL of a 100 × protease inhibitor mixture (GE Healthcare), and further mixed with 20 μL of 100 × lysozyme (Clontech, CA, USA). The resulting mixture was incubated at 37°C for five minutes, and then sonicated with ice cooling. A pellet (inclusion body) containing Strep-tag-fused VEGF164 collected by centrifugation of the sonicated solution at 4°C at 20000 g for 15 minutes was washed three times with a buffer containing 100 mM Tris-HCl pH 7.5, 5 mM EDTA pH 8.0, 10 mM DTT, 2 mM urea, and 0.2% (w/v) Triton-X100. The inclusion body was washed three more times with a buffer containing 100 mM Tris-HCl pH 7.5, 5 mM EDTA pH 8.0, and 10 mM DTT.

• Purification and collection of expressed protein by solubilization of inclusion body

The inclusion body was solubilized with a 2 mL guanidine solution containing 6 M guanidine hydrochloride, 0.1 M $NaH_2PO_4$, and 10 mM Tris-HCl (pH 8.5). The solubilized inclusion body was dialyzed using a 400 mL urea solution containing 6 M urea, 0.1 M $Na_2PO_4$, and 10 mM Tris-HCl (pH 8.0) at 4°C for six hours, subsequently dialyzed using a urea solution having a similar composition for four hours, and further dialyzed using an exchanged urea solution for 12 hours. A urea solution containing a Strep-tag-fused VEGF monomer was thereby prepared.

Note that the dialysis was performed using a dialysis membrane: Spectra/Por (registered trademark) 6 (fraction molecular weight: 2000, Spectrum Lab Japan).

The Strep-tag-fused VEGF164 solution after the dialysis was adjusted to 0.5 mg/mL with the urea solution. The protein quantification of the solution was used by measuring absorbance using a Coomassie (Bradford) protein assay kit (Thermo Fisher Scientific K.K.) with a spectrophotometer U-4100 (manufactured by Hitachi High-Technologies Corporation).

[Production Example 4]

<His-tag-fused VEGF subunit>

DNA was amplified and purified in a similar manner to Production Example 1 except that His-tag5'-primer (5'-CATCACCATTAATGAGCTTGACCTGTGAAGTGAAAA-3') was used instead of 5'-primer, and His-tag3'-primer (5'-GTGATGGTGAGCGCTCTCGAGCCGCCTTGGCTTGTC-3') was used instead of 3'-primer. In the purified PCR product, Strep-Tag was converted into His-Tag. Using Mighty Cloning Reagent Set-Blunt End (manufactured by Takara Bio Inc.), 200 ng of the PCR product of the purified His-tag-fused VEGF was used as cDNA.

Migration and purification of cDNA were performed in a similar manner to Production Example 1. Furthermore, BL21 Rosetta (registered trademark) 2 (DE3) competent cells (Merck Millipore) were transformed in a similar manner. Expression, purification, and collection of a His-tag-fused VEGF monomer were thereby performed.

[Production Example 5]

<Streptavidin-bonded perylenediimide-integrated melamine resin particles>

N,N'-bis(2,6-diisopropylphenyl)-1,6,7,12-tetraphenoxyperylene-3,4:9,10-tetracarboxydiimide was treated with concentrated sulfuric acid to manufacture a perylenediimide sulfonic acid derivative. The perylenediimide sulfonic acid derivative was converted into an acid chloride to obtain a perylene diimide sulfonic acid chloride derivative. In 22.5 mL of pure water, 17.3 mg of the perylene diimide sulfonic acid chloride derivative was dissolved. Thereafter, the resulting mixture was stirred for 20 minutes while the temperature of the solution was maintained at 70°C with a hot stirrer. To the solution after stirring, 0.78 g of melamine resin "Nikarac MX-035" (Nippon Carbide Industrial Co., Ltd.) was added, and the resulting mixture was further heated and stirred under the same conditions for five minutes. To the mixed liquid after stirring, 100 $\mu$L of formic acid was added, and the resulting mixture was stirred for 20 minutes while the temperature of the mixed liquid was maintained at 60°C. Thereafter, then the mixed liquid was left and cooled to room temperature. The reaction mixed liquid after cooling was injected into a centrifuge tube, and centrifuged at 12,000 rpm for 20 minutes to precipitate perylenediimide-integrated melamine resin particles contained in the mixed liquid. The supernatant was removed, and the precipitated perylenediimide-integrated melamine resin particles were washed with ethanol and water. The average particle diameter of the perylenediimide-integrated melamine resin particles (average value of particle diameters of 100 particles counted in an SEM image) was 135 nm.

In 1.5 mL of EtOH, 0.1 mg of the perylenediimide-integrated melamine resin particles were dispersed, and 2 $\mu$L of aminopropyltrimethoxysilane "LS-3150" (Shin-Etsu Chemical Co., Ltd.) was added thereto to cause a reaction for eight hours, thus performing a surface amination treatment.

Subsequently, the particles that had been subjected to the surface amination treatment were adjusted so as to have a concentration of 3 nM using phosphate buffered saline (PBS) containing 2 mM ethylenediaminetetraacetic acid (EDTA). Succinimidyl-[(N-maleimidopropionamido)-dodecaethyleneglycol] ester (SM(PEG)$_{12}$, Thermo Scientific Co., Ltd.) was mixed with this solution so as to have a final concentration of 10 mM, and a reaction was caused for one hour. The mixed liquid was centrifuged at 10,000 G for 20 minutes, and the supernatant was removed. Thereafter, PBS containing 2 mM EDTA was added thereto to disperse the precipitate, and centrifugation was performed again. Washing by a similar procedure was performed three times to obtain maleimide-modified perylenediimide-integrated melamine resin particles.

Meanwhile, streptavidin (Wako Pure Chemical Industries, Ltd.) was subjected to a thiol group addition treatment using N-succinimidyl S-acetylthioacetate (SATA), and then filtration through a gel filtration column was performed to obtain a streptavidin solution that can be bonded to maleimide-modified perylenediimide-integrated melamine resin particles.

The maleimide-modified perylenediimide-integrated melamine resin particles and the streptavidin to which a thiol group had been added by the treatment were mixed in PBS containing 2 mM EDTA, and a reaction was caused at room temperature for one hour. Thereafter, 10 mM mercaptoethanol was added thereto, and the reaction was stopped. The obtained mixed liquid was concentrated using a centrifugal filter. Thereafter, streptavidin that had not reacted and the like were removed using a gel filtration column for purification to obtain streptavidin-modified perylenediimide-integrated melamine resin particles.

[Example 2]

<Production of Strep-tag/His-tag-fused VEGF>

A dimeric protein including the Strep-tag-fused VEGF164 and the His-tag-fused VEGF164 produced in Production Examples 2 and 3 as subunits was produced by the following method.

A solution containing 100 mM Tris-HCl (pH 8.5), 10 mM cysteine, 2 mM cystine, 0.5 M guanidine hydrochloride, and 0.5 M arginine hydrochloride was prepared and used as a buffer for refolding.

The Strep-tag-fused VEGF164 and the His-tag-fused VEGF164 produced in Production Examples 1 and 2 were mixed at a molar ratio of 1:1 and dialyzed at 37°C for six hours using a 50-fold amount of a buffer for refolding, dialyzed using an exchanged buffer for four hours, and further dialyzed using an exchanged buffer for 12 hours.

The liquid after the dialysis was dialyzed at 4°C for six hours using a 50-fold amount of 0.1 M Tris-HCl (pH 7.5) buffer, dialyzed using an exchanged buffer for four hours, and further dialyzed using an exchanged buffer for 12 hours.

The refolded protein sample was purified using a His-tag affinity column (HisTALON (trademark) Superflow Cartridge, Takara Bio Inc.) and (Strep-Tactin (trademark) Superflow high-capacity cartridge, IBA). Purification was performed by a procedure according to each manual.

The purified product was concentrated by centrifugation at 5000 g at 4°C to less than 1 ml using an Amicon Ultra centrifugal filter unit (fraction molecular weight: 3,000, Merck Millipore).

Furthermore, dialysis was performed at 4°C for six hours using 100 ml of PBS, further dialyzed using exchanged PBS for four hours, and further dialyzed using exchanged PBS for 12 hours.

After the dialysis, the same amount of glycerol was added thereto to adjust the concentration to 50 ng/$\mu$L.

For the glycerol solution, western blotting was performed using a rabbit anti-mouse VEGF polyclonal primary antibody (manufactured by Abcam), a rabbit anti-Strep-tag polyclonal antibody (manufactured by Abnova), and a rabbit anti-His-tag polyclonal antibody (Abcam), and it was confirmed that the reaction had occurred with any one of the anti-VEGF antibody, the anti-His-tag antibody, and the anti-strep-tag antibody. This means that the glycerol solution contains the target dimeric protein, that is, Strep-tag/His-tag-fused VEGF (dimeric protein of the present invention) including Strep-tag-fused VEGF164 and His-tag-fused VEGF164 as subunits.

[Example 3]

<Staining of cell with fluorescence labelling probe>

The Strep-tag/His-tag-fused VEGF produced in Example 1 and Qdot (registered trademark) 705 streptavidin conjugate (hereinafter, also referred to as QD705) (Thermo Fisher Scientific K.K.) were mixed at a molar ratio of 8:1. The resulting mixture was allowed to stand at 25°C for one hour to produce a fluorescence labelling probe.

Mouse pancreas-derived cultured cell lines MS1 (obtained from ATCC) were seeded and cultured in a 96-well plate using 5% FBS-added DMEM. One day after seeding, the above fluorescence labelling probe was added to the wells so as to have a concentration of 10 nM. The resulting mixture was allowed to stand at 10°C for one hour, and then washed with PBS. A round cover glass was placed on the wells to prepare a specimen slide. The specimen slide was set on a microscope, and a fluorescence image was captured and processed. Note that cells were treated only using QD705 as a control, or using a VEGF dimer (not biotin-modified) and QD705, and a fluorescence image was captured and processed similarly.

For irradiation with excitation light and observation of emission of fluorescence in fluorescence observation, an inverted fluorescence microscope IX71 (Olympus Optical Industry Co., Ltd.) connected to a confocal scanner unit CSU (Yokogawa Electric Corporation) was used. For imaging, an EMCCD camera iXon DV887 (Andor) attached to the fluorescence microscope was used.

First, the specimen slide was irradiated with excitation light corresponding to perylenediimide to emit fluorescence, and a fluorescence image in this state was captured. At this time, the wavelength of the excitation light was set to 532 nm using an optical filter for excitation light included in the fluorescence microscope, and the wavelength of the fluorescence to be observed was set to 695 to 740 nm using an optical filter for fluorescence. The intensity of excitation light at the time of observation with a fluorescence microscope and image capturing was set such that irradiation energy near the center of a visual field was 900 W/cm$^2$. Exposure time at the time of image capturing was adjusted within a range where the brightness of an image was not saturated, and was set to, for example, 4000 $\mu$sec.

The fluorescence images of the perylenediimide fluorescence image were superimposed on each other by image processing using image processing software "Image J" (open source). For fluorescent intensity, an acquired image was converted into gray scale using the software "Image J", and cut out in an area of 250 pixels $\times$ 300 pixels. Background correction was performed. An average value of pixel values per pixel was calculated from an average value of pixel values in 100 visual fields, and multiplied by 1000 pixels to quantify the total fluorescent intensity.

Fig. 6 illustrates a graph of the fluorescent intensity quantified in each of the acquired fluorescence images. The fluorescent intensity measured in cells stained with the fluorescence labelling probe was $16 \times 10^3$ (a.u.), indicating that the cells could be detected with extremely high sensitivity as compared with the control.

[Comparative Example 2]

For mouse VEGF164 recombinant protein (R & D Systems), biotinylation was performed in a similar manner to Non Patent Literature 1 using a biotinylation kit EZ-Link (registered trademark) Micro Sulfo-NHS-LC-Biotinylation Kit (manufactured by Thermo Fisher Scientific K.K.). For the obtained biotinylated VEGF164, the average number of streptavidin bonding sites per mole of VEGF was measured using a Biotin Quantitation Kit (Thermo Fisher Scientific K.K.) and a spectrophotometer U-4100 (Hitachi High-Technologies Corporation), and found to be five.

fluorescence staining was performed using MS1 VEGF (obtained from ATCC), which is a cell in which VEGF generation is promoted by introducing genes into mouse pancreas-derived cultured cell lines MS1 and MSI cells in a similar manner to Example 2 except that the biotinylated VEGF164 was used instead of Strep-tag/His-tag-fused VEGF. Note that for cells that had been treated only with QD705 as a control, a fluorescence image was captured and processed similarly.

(Results and discussion)

In Comparative Example 1, bright spots were confirmed in a fluorescence image of MS1 -VEGF cells, which are VEGFR-highly expressing cells, but almost no bright spots were observed in MS1 cells. The fluorescent intensity is $9 \times 10^3$ (a.u.) in MS1 cells. This value is not largely different from a numerical value obtained in the control (QD705).

As described above in Example 3, in staining using the fluorescence labelling probe of the present invention, bright spots can be clearly observed even in MS1 cells having a low VEGFR expression level, and the difference in the fluorescent intensity as compared with the control was also significantly large.

From the above results, even in cells where VEGFR cannot be detected with biotinylated VEGF produced by a conventional method, VEGF can be detected by using the fluorescence labelling probe of the present invention. Therefore, this suggests that staining can be performed with higher sensitivity than the conventional method by using the fluorescence labelling probe of the present invention, and that more accurate drug evaluation can be performed by using the fluorescence labelling probe in the drug evaluation method of the present invention.

[Example 4]

<Drug evaluation using Strep-tag/His-tag-fused VEGF>

Three weeks and five weeks after cancer cell transplantation, bevacizumab (Avastin (registered trademark), Chugai Pharmaceutical Co., Ltd.) prepared with saline so as to have a concentration of 25 $\mu$g/$\mu$L was administered from the tail vein. The dose for a mouse body weight was 5 $\mu$g/g. The same amount of saline was administered to a cancer-bearing mouse similarly produced as a control. At each time point, body weights and tumor volumes of the mice were measured. A short axis (a) and a long axis (b) of a tumor were measured using a caliper, and the volume of the tumor was determined by the following formula.

$$\text{“Volume”} = (4/3)\ \pi a^2 \times b$$

Fig. 3 illustrates average values of body weights and tumor volumes at each time point. Suppression of tumor volume in a tumor cell area was not observed five weeks after the transplantation. This strongly suggests that a plurality of administrations of bevacizumab is effective.

After administration of bevacizumab three weeks, five weeks, or seven weeks after cancer cell transplantation, 2.5 $\mu$g of Strep-tag/His-tag-fused VEGF produced in Example 1 and 100 ug of Dylight488-labeled lectin (Vector Laboratories) were administered by tail vein injection. 45 minutes after the administration, the mouse was sacrificed, and a tissue containing a cancer cell was extracted. The tissue was fixed by formalin, dehydrated, embedded in paraffin, and cut into 100 to 400 $\mu$m sections to produce a tissue sample.

The tissue sample was subjected to a deparaffinization treatment and an activation treatment according to a usual method, and then immersed in a container containing PBS for 30 minutes and washed. Thereafter, the tissue sample was immersed in a 100 ug/mL casein solution at 4°C for one hour to perform blocking. After blocking, the tissue sample was washed again with PBS.

The tissue sample was immersed in a dispersion obtained by dispersing the streptavidin-bonded perylenediimide-integrated melamine resin nanoparticles produced in Production Example 4 in PBS so as to have a concentration of 0.1 nM at 4°C for two hours. Thereafter, the tissue sample was washed three times with PBS and placed on a cover glass. Furthermore, the specimen was fixed to a microscope with an aluminum plate and screws. A fluorescence image of perylenediimide was captured in a similar manner to Example 2. Next, the wavelength of excitation light was set to 488 nm, the wavelength of fluorescence to be observed was set to 500 to 540 nm, and a fluorescence image corresponding to Dylight488 was captured. The fluorescence image of perylenediimide and the fluorescence image of Dylight488 were processed in a similar manner to Example 2, and the fluorescent intensity was measured.

Fluorescence images at time points are illustrated in a to e of Fig. 7, and graphs of quantitative results are illustrated in g and f of Fig. 7.

(Results and discussion)

Table 1 illustrates results in the saline administration group. Five weeks after cancer cell transplantation, an increase in the expression level of the VEGF receptor was significantly observed in both the vascular endothelial area and the tumor cell area, as compared with the results three weeks after the cancer cell transplantation. Meanwhile, seven weeks after the transplantation, the expression level further increased in the vascular endothelial area, but hardly changed in the tumor cell area.

From these results, it is considered that the expression level of the VEGF receptor in the tumor cells increased first, angiogenesis was thereby induced, and the expression level of the receptor in the vascular endothelium increased next.

[Table 1]

|  | 3 weeks after transplantation | 5 weeks after transplantation | 7 weeks after transplantation |
|---|---|---|---|
| Vascular endothelial area | 1.0 | 1.7 | 3.3 |
| Tumor cell area | 1.0 | 2.0 | 2.2 |
| *A numerical value 3 weeks after cancer cell transplantation is assumed to be 1.0. | | | |

Table 2 illustrates results in the bevacizumab administration group. Five weeks after the transplantation, there was no significant difference in the expression level of the VEGF receptor in the vascular endothelial area, while the expression level of the VEGF receptor increased in the tumor cell area with a significant difference. Meanwhile, seven weeks after the transplantation, no significant change was observed in the expression level of the VEGF receptor in the vascular endothelial area, but the expression level was reduced in the tumor cell area with a significant difference.

[Table 2]

|  | 3 weeks after transplantation | 5 weeks after transplantation | 7 weeks after transplantation |
|---|---|---|---|
| Vascular endothelial area | 1.0 | 1.3 | 1.1 |
| Tumor cell area | 1.0 | 1.4 | 0.8 |
| *A numerical value 3 weeks after cancer cell transplantation is assumed to be 1.0. | | | |

When the results are compared between the groups, in the expression level of the VEGF receptor five weeks after the cancer cell transplantation, there was no significant difference between the saline administration group and the bevacizumab administration group in the vascular endothelial area. However, in the tumor cell area, it was found that the expression level of the VEGF receptor was significantly suppressed in the bevacizumab administration group (0.70 times). In addition, it was found that the expression level of the VEGF receptor seven weeks after the transplantation was significantly suppressed in both the tumor cell area (0.36 times) and the vascular endothelial area (0.33 times) in the bevacizumab administration group as compared with the saline administration group.

[Example 5]

<Physiological activity evaluation of Strep-tag/His-tag-fused VEGF>

$2.0 \times 10^3$ mouse pancreas-derived cultured cell lines MS1 (obtained from ATCC) were seeded in a 96-well plate and cultured using 5% FBS-added DMEM. One day after seeding, the 5% FBS-added DMEM was exchanged with a medium obtained by adding Strep-tag/His-tag-fused VEGF produced in Example 1 or mouse VEGF164 recombinant protein (R & D Systems) as a control to 5% FBS-added DMEM so as to have a concentration of 0, 0.1, 1, 10, or 100 pg/mL. Four days after seeding, a cell growth test was performed using a CellTiter 96 (registered trademark) AQueous One Solution cell growth test kit (Promega Corporation).

Fig. 8 illustrates results thereof. There was no significant difference in absorbance between the case where mouse VEGF164 recombinant protein was added (white) and the case where Strep-tag/His-tag-fused VEGF was added (gray). This indicates that, like mouse VEGF164 recombinant protein, Strep-tag/His-tag-fused VEGF can specifically retain bonding ability to the VEGF receptor and can activate growth ability of VEGF receptor-expressing cells, and that localization of the VEGF receptors and the amount thereof on cell membranes can be measured. Therefore, it is considered that Strep-tag/His-tag-fused VEGF can be used for physiological evaluation such as localization of the VEGF receptors and the amount thereof on cell membranes.

[Example 6]

<Drug evaluation using gold nanoparticles and Strep-tag/His-tag-fused VEGF>

The following table summarizes the results of Examples 1 and 3 to 5 described above. The tumor volume, the blood vessel volume, and the blood vessel volume/tumor volume indicate the results of Example 1, and VEGFR expression level (tumor area) and VEGFR expression level (vascular endothelial area) indicate the result of Examples 3 to 5.

[Table 3]

|  |  | 3 weeks after transplantation | 5 weeks after transplantation | 7 weeks after transplantation |
|---|---|---|---|---|
| Tumor volume | Con. | 1.0 | 4.8 | 19.2 |
|  | BV. | 1.0 | 3.8 | 7.6 |
| Blood vessel volume | Con. | 1.0 | 6.4 | 49.9 |
|  | BV. | 1.0 | 5.0 | 14.0 |
| Blood vessel volume/Tumor volume | Con. | 1.0 | 1.4 | 2.7 |
|  | BV. | 1.0 | 1.4 | 1.6 |
| VEGFR expression level (tumor area) | Con. | 1.0 | 2.0 | 2.2 |
|  | BV. | 1.0 | 1.4 | 0.8 |
| VEGFR expression level (vascular endothelial area) | Con. | 1.0 | 1.7 | 3.3 |
|  | BV. | 1.0 | 1.3 | 1.1 |
| Con.: Control (saline) administration group, BV: bevacizumab administration group *A numerical value 3 weeks after cancer cell transplantation is assumed to be 1.0. | | | | |

(Discussion)

Here, by combining the drug evaluation performed using the image acquired in the imaging step (1) (Example 1) and the drug evaluation performed using the image acquired in the imaging step (2) (Examples 3 to 5), the following is suggested.

As described above, in Example 1, three to five weeks after the cancer cell transplantation, there was no large difference in the tumor volume, the blood vessel volume, and the ratio of the blood vessel volume between the bevacizumab administration group and the saline administration group. However, five to seven weeks after the transplantation, it was found that an increase in the tumor volume, the blood vessel volume, and the ratio of the blood vessel volume was suppressed in the bevacizumab administration group as compared with the saline administration group.

Meanwhile, in the bevacizumab administration group, significant suppression of the VEGFR expression level was observed in the tumor cell area five weeks after the transplantation, but no significant difference was observed in the vascular endothelial area. That is, at this stage, it can be said that bevacizumab remains in tumor cells, and an effect on the VEGFR expression level of the vascular endothelial cells is small. This causes the result of Example 1. That is, there is no significant difference in the tumor volume, and the blood vessel volume and the ratio of the blood vessel volume to the tumor volume, which is the blood vessel information acquired in the imaging step (1), at this time point.

Furthermore, seven weeks after the transplantation, in the bevacizumab administration group, the VEGFR expression level was significantly suppressed in both the tumor cell area and the vascular endothelial area.

This suggests that a VEGFR expression inhibitory effect of bevacizumab on tumor cells may also spread to vascular endothelial cells and suppress VEGF-VEGFR signaling in blood vessels. It is considered that this may cause the result of Example 1, that is, significant suppression of the tumor volume, and the blood vessel volume and the ratio of the blood vessel volume to the tumor volume, which is the blood vessel information acquired in the imaging step (1), in the bevacizumab administration group.

In other words, the following is strongly suggested. That is, three to five weeks after the cancer cell transplantation, the increase in the VEGFR expression level in the tumor cell area is mainly related to the increase in the blood vessel volume and the ratio of the blood vessel volume to the tumor volume, and five to seven weeks after the transplantation, the increase in the VEGFR expression level in the vascular endothelial area is mainly related to the increase in the blood vessel volume and the ratio of the blood vessel volume to the tumor volume.

In addition, five to seven weeks after the transplantation, the VEGFR change amount in the tumor cell area was larger than that in the vascular endothelial cell area. Therefore, it is considered that the expression level of VEGFR in the tumor cell area affects the VEGFR expression level in the vascular endothelial area and this leads to a superior change in the blood vessel volume and the ratio of the blood vessel volume to the tumor volume.

As described above, by an image acquired using the gold nanoparticles corresponding to the imaging step (1) of the present invention, it is possible to acquire more detailed information including blood vessel information than a conventional technique, and by an image acquired using Strep-tag/His-tag-fused VEGF corresponding to the imaging step (2) of the present invention, VEGFR can be detected with higher sensitivity.

As a result, it is considered that by combining evaluation based on information including blood vessel information and evaluation based on the expression level of VEGFR, more accurate drug evaluation can be performed. In addition, it may be possible to judge whether an anticancer effect of the drug is based on an angiogenesis inhibitory effect of VEGF in tumor cells. Therefore, the evaluation is also useful in drug evaluation when screening is performed on a new anti-VEGF candidate drug.

Reference Signs List

1    Fluorescence observation device
10   Radiography device
20   Information processing device
25   Display device
30   Input device
31   Controller
33   Reflection image generator
36   Fluorescence image generator
50   Light source
51   Mirror
52   Half mirror
55   Filter
60   Lens
70   Sample
80   Stage

**Claims**

1. A drug evaluation method to be performed using an animal having a lesion as a subject, the method comprising:

   a drug administration step (A) of administering a drug involved in angiogenesis to the subject;
   further performing the following imaging step (1) a plurality of times, at least one of which is performed after the drug administration step (A), or performing the following imaging step (2); and
   further evaluating the drug using an image acquired in the imaging step (1) or (2), wherein
   in the imaging step (1), radiography is performed on a subject in a state where metal nanoparticles are present in a blood vessel, and
   in the imaging step (2), using a dimeric protein containing a first monomer which is a fusion peptide fused to a tag bonded to streptavidin at one end, and a second monomer,
   at least two tissue sections are obtained from among a tissue section collected from the subject before the drug administration step (A), a tissue section collected from the subject at a first time point after the drug administration step (A), and a tissue section collected from the subject at a second time point which is a time point after the first time point, each of the sections is fluorescently stained, and each of the fluorescently stained samples is imaged.

2. The drug evaluation method according to claim 1, wherein the dimeric protein contains a first monomer which is a fusion peptide fused to a tag bonded to streptavidin at one end, and a second monomer fused to a tag bonded to a protein other than streptavidin at one end.

3. The drug evaluation method according to claim 1 or 2, wherein the second monomer is a fusion peptide fused to a tag bonded to a protein to other than streptavidin only at one end.

4. The drug evaluation method according to any one of claims 1 to 3, wherein both the first monomer and the second monomer are linear.

5. The drug evaluation method according to any one of claims 1 to 4, wherein
   a peptide sequence other than the tag bonded to streptavidin in the first monomer has the same sequence as a peptide sequence other than the tag bonded to a protein other than streptavidin in the second monomer.

6. The drug evaluation method according to any one of claims 1 to 5, wherein the dimeric protein is derived from a vascular endothelial cell growth factor (VEGF) or a platelet-derived growth factor (PDGF).

7. The drug evaluation method according to any one of claims 1 to 6, wherein the fluorescence staining is a method for staining a target protein to be specifically bonded to the dimeric protein by bonding the dimeric protein to a fluorescent label.

8. The drug evaluation method according to any one of claims 1 to 6, wherein the fluorescence staining is a method for staining a target protein to be specifically bonded to the dimeric protein by bonding the target protein to a fluorescence labelling probe in which a fluorescent label is bonded to the dimeric protein.

9. The drug evaluation method according to claim 1, wherein the metal nanoparticles are gold nanoparticles.

10. The drug evaluation method according to claim 7 or 8, wherein the fluorescent label is a fluorescent dye, a quantum dot, or a fluorescent dye-integrated nanoparticle bonded to streptavidin.

11. The drug evaluation method according to any one of claims 1 to 10, wherein in the imaging step (2), fluorescent dye staining which is staining using a fluorescent dye is further performed.

12. The drug evaluation method according to claim 11, wherein the fluorescent dye staining is staining specific to a vascular endothelial cell.

13. The drug evaluation method according to any one of claims 1 to 12, wherein in the imaging step (2), dye staining with a dye and bright field imaging are further performed.

14. A dimeric protein comprising a first monomer which is a fusion peptide fused to a tag bonded to streptavidin at one end, and a second monomer.

15. A dimeric protein comprising a first monomer which is a fusion peptide fused to a tag bonded to streptavidin at one end, and a second monomer fused to a tag bonded to a protein other than streptavidin at one end.

16. The dimeric protein according to claim 14 or 15, wherein both the first monomer and the second monomer are linear.

17. The dimeric protein according to claim 15, wherein
a peptide sequence other than the tag bonded to streptavidin in the first monomer has the same sequence as
a peptide sequence other than the tag bonded to a protein other than streptavidin in the second monomer.

18. The dimeric protein according to any one of claims 14 to 17, derived from a vascular endothelial cell growth factor (VEGF) or a platelet-derived growth factor (PDGF).

19. A method for producing a dimeric protein, comprising:

a purification step of mixing
a first monomer which is a fusion peptide fused to a tag bonded to streptavidin only at one end, and
a second monomer which is a fusion peptide fused to a tag bonded to a protein to other than streptavidin only at one end; and
purifying a dimeric protein containing the first monomer and the second monomer by removing contaminants from the obtained mixture.

20. The method for producing a dimeric protein according to claim 19, wherein the purification step includes a first purification step of performing purification using a first purification column carrying a carrier to be specifically bonded to a tag fused to the first monomer, and a second purification step of performing purification using a second purification column carrying a carrier to be specifically bonded to a tag fused to the second monomer.

21. A fluorescence labelling probe in which the fluorescent label is bonded to the dimeric protein according to any one of claims 14 to 18.

22. The fluorescence labelling probe according to claim 21, wherein the fluorescent label is a fluorescent dye, a quantum dot, or a fluorescent dye-integrated nanoparticle bonded to streptavidin.

23. A fluorescence staining liquid comprising the fluorescence labelling probe according to claim 21 or 22.

24. A fluorescent labeling and staining method for staining a target protein to be specifically bonded to the dimeric protein containing a first monomer and a second monomer by bonding a fluorescent label to the dimeric protein according to any one of claims 14 to 18, wherein
the fluorescent label is a fluorescent dye, a quantum dot, or a fluorescent dye-integrated nanoparticle bonded to streptavidin.

25. The fluorescent labeling and staining method according to claim 24, further comprising fluorescent dye staining.

26. The fluorescent labeling and staining method according to claim 24 or 25, wherein the dye staining is staining specific to a vascular endothelial cell.

27. A physiological activity evaluation method comprising the staining method according to any one of claims 24 to 26.

28. The drug evaluation method according to any one of claims 1 to 13, wherein the drug is evaluated by acquiring information including blood vessel information from an image obtained in the imaging step (1) or (2).

29. The drug evaluation method according to claim 28, wherein the blood vessel information includes information on a volume of a blood vessel in the lesion.

30. The drug evaluation method according to claim 28 or 29, wherein the blood vessel information includes positional information of a blood vessel in the lesion.

**31.** The drug evaluation method according to any one of claims 1 to 13 and 28 to 30, wherein the information further includes pathological information.

**32.** The drug evaluation method according to claim 31, wherein the pathological information includes information on a volume of the lesion.

**33.** The drug evaluation method according to claim 31 or 32, wherein the pathological information includes positional information of a lesion.

**34.** The drug evaluation method according to any one of claims 1 to 13 and 28 to 33, wherein the lesion is a tumor part.

**35.** The drug evaluation method according to any one of claims 1 to 13 and 28 to 34, wherein the drug is involved in angiogenesis.

**36.** The drug evaluation method according to any one of claims 1 to 13 and 28 to 35, wherein the drug is an angiogenesis inhibitor.

**37.** The drug evaluation method according to any one of claims 28 to 36, wherein
the information including blood vessel information includes information on a volume of a blood vessel in the lesion and a volume of the lesion, and
a value obtained by dividing the volume of the blood vessel in the lesion by the volume of the lesion is calculated, and a change in the value is observed.

**38.** The drug evaluation method according to any one of claims 1 to 13 and 28 to 37, wherein the radiography in the imaging step (1) is three-dimensional radiography, and the imaging in the imaging step (2) is fluorescence imaging.

**39.** The drug evaluation method according to any one of claims 1 to 13 and 28 to 38, wherein the subject is an experimental animal.

**40.** The drug evaluation method according to any one of claims 1 to 13 and 28 to 39, wherein the subject is a cancer-bearing mouse.

**41.** A drug evaluation system for performing the drug evaluation method according to any one of claims 1 to 13 and 28 to 40, the drug evaluation system comprising
an imaging device including at least one of a radiography device and a fluorescence imaging device, and an information processing device, wherein
the information processing device
receives an image acquired by the radiography device or the fluorescence imaging device, and
evaluates a drug involved in angiogenesis using the received image.

**42.** The drug evaluation system according to claim 41 for performing the drug evaluation method according to any one of claims 1 to 13 and 28 to 40, the drug evaluation system comprising
an imaging device including at least one of a radiography device and a fluorescence imaging device, and an information processing device, wherein
the information processing device
receives an image acquired by the radiography device or the fluorescence imaging device, and
further evaluates a drug involved in angiogenesis using information including blood vessel information acquired from the received image.

**43.** The drug evaluation system according to claim 41 or 42, wherein the imaging device further includes a device for performing bright field imaging.

**44.** The drug evaluation system according to any one of claims 41 to 43, further comprising a display device that displays at least one of the image, the information including blood vessel information, the analysis result, and drug evaluation.

**45.** The drug evaluation system according to any one of claims 41 to 44, wherein the information processing device further includes an information storage device that stores at least one of the image, the information including blood vessel information, the analysis result, and drug evaluation as data.

46. A program for causing a computer to execute the drug evaluation method according to any one of claims 1 to 13 and 28 to 40.

# FIG. 1

```
                    ┌─────────────────┐
                    │     START       │
                    └────────┬────────┘
                             ↓
              ┌────────────────────────────┐
              │       INPUT MOUSE ID        │
              └──────────────┬─────────────┘
                             ↓
          ┌────────────────────────────────────┐
          │    INJECT METAL NANOPARTICLES       │
          └─────────────────┬──────────────────┘
                            ↓
              ┌────────────────────────────┐
              │          IMAGING            │
              └──────────────┬─────────────┘
                             ↓
              ┌────────────────────────────┐
              │     ACQUIRE INFORMATION     │
              └──────────────┬─────────────┘
                             ↓
              ┌────────────────────────────┐
              │        INPUT DRUG ID        │
              └──────────────┬─────────────┘
                             ↓
              ┌────────────────────────────┐
              │       ADMINISTER DRUG       │◄────────────┐
              └──────────────┬─────────────┘             │
                             ↓                            │
          ┌────────────────────────────────────┐         │
          │    INJECT METAL NANOPARTICLES       │◄────┐   │
          └─────────────────┬──────────────────┘     │   │
                            ↓                          │   │
              ┌────────────────────────────┐          │   │
              │          IMAGING            │          │   │
              └──────────────┬─────────────┘          │   │
                             ↓                          │   │
              ┌────────────────────────────┐          │   │
              │     ACQUIRE INFORMATION     │──────────┴───┘
              └──────────────┬─────────────┘
                             ↓
              ┌────────────────────────────┐
              │         EVALUATION          │
              └──────────────┬─────────────┘
                             ↓
                    ┌─────────────────┐
                    │      END        │
                    └─────────────────┘
```

## FIG. 2

a

Saline treatment Group

Bulb/c nude mouse 6W Female

SKOV3
$50 \times 10^6$

3w after injection

treated by Saline

5w after injection

treated by Saline

7w after injection

Bevacizumab treatment Group

3w after injection

treated by Bevacizumab

5w after injection

treated by Bevacizumab

7w after injection

## FIG. 3

a

Body weight (g)

— Saline
···· Bevacizumab

30

25

0

3w    5w    7w

b  $(\times 10^2)$

Tumor volume $(mm^3)$

— Saline
···· Bevacizumab

10

5

0

3w    5w    7w

\*

# FIG. 4

treated by saline

treated by Bevacizumab

## FIG. 5

## FIG. 6

## FIG. 7

FIG. 8

# FIG. 9

(A)

DISPLAY DRUG ID: B45539
MOUSE ID: 2017267
JUDGEMENT RESULT: RANKB
CHANGE AMOUNT: 63%
MEASUREMENT DATE AND TIME:   1: 201706080600
                             2: 201706300600
                             3: 201707080600

25

20

10

30

(B)

50

51

52

55

60

70

80

31

33

36

20

25

ID: B45539          JUDGMENT: RANKB
Date: 20170627

NUMBER OF BRIGHT SPOTS   NUMBER OF BRIGHT SPOTS
BRIGHTNESS               BRIGHTNESS

30

1

## INTERNATIONAL SEARCH REPORT

| International application No. |
| --- |
| PCT/JP2018/042826 |

**A. CLASSIFICATION OF SUBJECT MATTER**
Int.Cl. G01N33/48(2006.01)i,     A61B6/00(2006.01)i,     A61B6/03(2006.01)i,
        A61K49/04(2006.01)i

According to International Patent Classification (IPC) or to both national classification and IPC

**B. FIELDS SEARCHED**

Minimum documentation searched (classification system followed by classification symbols)
Int.Cl. G01N33/48, A61B6/00, A61B6/03, A61K49/04

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
PubMed

**C. DOCUMENTS CONSIDERED TO BE RELEVANT**

| Category* | Citation of document, with indication, where appropriate, of the relevant passages | Relevant to claim No. |
| --- | --- | --- |
| X | JP 2012-519902 A (BIO-TREE SYTEMS, INC.) 30 August 2012, claims, paragraph [0260], examples 8-9 & US 2012/0150048 A1 (claims, paragraph [0317], examples 8-9) & WO 2010/101660 A2 & EP 2429399 A2 | 1-13, 28-46 |
| X | HAMADA, Y. et al., "In vivo imaging of the molecular distribution of the VEGF receptor during angiogenesis in a mouse model of ischemia", BLOOD, 29 September 2011, vol. 118, no. 13, e93-e100 | 14, 16, 18, 21-27 |
| Y | | 15, 17, 19-20 |

☒ Further documents are listed in the continuation of Box C.       ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 29 January 2019 (29.01.2019) | 12 February 2019 (12.02.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/042826

C (Continuation). DOCUMENTS CONSIDERED TO BE RELEVANT

| Category* | Citation of document, with indication, where appropriate, of the relevant passages | Relevant to claim No. |
|---|---|---|
| X | LEUNG, K., "Biotinylated vascular endothelial growth factor121-Avi-streptavidin-IRDye800", | 14, 16, 18, 21-27 |
| Y | MOLECULAR IMAGING AND CONTRAST AGENT DATABASE, 07 July 2009, 1-5 | 15, 17, 19-20 |
| X | JP 2014-531424 A (INDI MOLECULAR, INC.) 27 November 2014, example 8 & US 2013/0156692 A1 (example 8) & | 14, 16, 18, 21-27 |
| Y | WO 2013/033561 A1 & EP 2751135 A1 | 15, 17, 19-20 |
| Y | JP 2017-532045 A (HALOZYME, INC.) 02 November 2017, paragraph [0371] & US 2018/0216095 A1 & WO 2016/061286 A2 (page 149, lines 7-30) & EP 3207130 A2 | 15, 17, 19-20 |
| A | US 2012/0071406 A1 (VEGENICS PTY LIMITED) 22 March 2012, entire text, all drawings & WO 2007/022287 A2 & EP 1919944 A2 | 1-46 |
| A | JP 2006-523314 A (MONOGRAM BIOSCIENCES, INC.) 12 October 2006, entire text, all drawings & WO 2004/091384 A2 | 1-46 |

Form PCT/ISA/210 (continuation of second sheet) (January 2015)

## INTERNATIONAL SEARCH REPORT

| International application No. |
| --- |
| PCT/JP2018/042826 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
|---|
| PCT/JP2018/042826 |

(Continuation of Box III)

Document 1: JP 2012-519902 A (BIO-TREE SYTEMS, INC.) 30 August 2012, claims, paragraph [0260], examples 8-9 & US 2012/0150048 A1 (claims, paragraph [0317], examples 8-9) & WO 2010/101660 A2 & EP 2429399 A2
Document 2: HAMADA, Y. et al., "In vivo imaging of the molecular distribution of the VEGF receptor during angiogenesis in a mouse model of ischemia", BLOOD, 29 September 2011, vol. 118, no. 13, e93-e100
Document 3: LEUNG, K., "Biotinylated vascular endothelial growth factor121-Avi-streptavidin-IRDye800", MOLECULAR IMAGING AND CONTRAST AGENT DATABASE, 07 July 2009, 1-5
Document 4: JP 2014-531424 A (INDI MOLECULAR, INC.) 27 November 2014, example 8 & US 2013/0156692 A1 (example 8) & WO 2013/033561 A1 & EP 2751135 A1

(Invention 1) Claims 1-3 and 28-46
Document 1 discloses a "method for evaluating a drug related to angiogenesis in which radiography is performed on a subject to be examined in a state in which metal nanoparticles are present in a blood vessel," and the invention in claim 1 in which an imaging process (1) is selected and which is the first option in claim 1 lacks novelty in light of document 1, and thus does not have a special technical feature.
However, claim 3 dependent on claim 1 has the special technical feature of a "drug evaluation method in which a value obtained by dividing the volume of a blood vessel in a lesioned part by the volume of the lesioned part is calculated and changes in said value are observed" and claims 38-46 also have the same technical feature as claim 37. Thus, claims 1-13 and 28-46 are classified as invention 1.

(Invention 2) Claims 14-27
Claims 14-27 cannot be said to have an identical or corresponding technical feature to claims 1-13 and 28-46 classified as invention 1.
In addition, claims 14-27 are not substantially identical or corresponding to any of the claims classified as invention 1.
Thus, claims 14-27 cannot be classified as invention 1.
In addition, claims 14-27 have the special technical feature of a dimer protein composed of a first monomer that is a fusion peptide, one end of which is fused with a tag binding to streptavidin, and a second monomer (said dimer protein is a well-known feature as exemplified in documents 2-4, and a special technical feature is not found in claims 14-27), and are thus classified as invention 2.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007526756 A
- US 20080294035 A

**Non-patent literature cited in the description**

- **NAKAGAWA et al.** *Science and Technology of Advanced Materials,* 2016, vol. 17 (1), 387-397
- **QUAN-YU et al.** *Investigative Radiology,* December 2007, vol. 42 (12), 797-806
- **HAMADA et al.** *BLOOD,* 29 September 2011, vol. 118 (13), e93-100
- **KOHRT et al.** Defining the optimal murine models to investigate immune checkpoint blockers and their combination with other immunotherapies. *Annals of Oncology,* 2016, vol. 27 (7), 1190-1198